# EUROPEAN PATENT APPLICATION

(11) **EP 4 379 060 A1**
(43) Date of publication of application: **05.06.2024**
(21) Application number: 22210258.4
(22) Date of filing: 29.11.2022
(51) Int. Cl.: C12P 17/12, C12P 7/42, C12N 9/02, C12N 9/10, C12N 9/88

(54) **HIGH-EFFICIENCY PRODUCTION OF HYDROXYECTOINE**

(71) Applicant: Universität des Saarlandes, 66123 Saarbrücken (DE)
(72) Inventor: Christoph, Wittmann, 66740 Saarlouis (DE); Hoffmann, Sarah-Lisa, 69124 Eppelheim (DE); Jungmann, Lukas, 66125 Saarbrücken (DE); Lang, Caroline, 61169 Friedberg (DE)
(74) Representative: Schiweck Weinzierl Koch Patentanwälte Partnerschaft mbB

(57) **Abstract**

The present invention means and methods which allow the advantageous production of hydroxyectoine in a larger scale which is more efficient and less time and cost intensive. In particular the present invention provides in a first aspect a method for producing hydroxyectoine, comprising (a) providing a biocatalyst, wherein said biocatalyst is a recombinant bacterial host cell transformed with a promoter operably linked to a nucleotide sequence encoding an ectoine hydroxylase - ectD - (EC 1.14.11) followed by a transcriptional terminator, (b) providing a carbon source and ectoine, and (c) cultivating the bacterial host cell under suitable conditions to allow biocatalytic conversion of ectoine into hydroxyectoine, and thereby secreting the produced hydroxyectoine. The invention further relates to a recombinant bacterial host cell comprising a heterologous promoter operably linked to a nucleotide sequence encoding an ectoine hydroxylase - ectD - (EC 1.14.11) followed by a transcriptional terminator; the use of the recombinant bacterial host cell of the present invention for the production of hydroxyectoine; and a kit comprising a bacterial host cell of and a further bacterial host cell of the present invention for use in a method of for producing hydroxyectoine.

## Description

### TECHNICAL FIELD OF THE INVENTION

The present invention means and methods which allow the advantageous production of hydroxyectoine in a larger scale which is more efficient and less time and cost intensive. In particular the present invention provides in a first aspect a method for producing hydroxyectoine, comprising (a) providing a biocatalyst, wherein said biocatalyst is a recombinant bacterial host cell transformed with a promoter operably linked to a nucleotide sequence encoding an ectoine hydroxylase - ectD - (EC 1.14.11) followed by a transcriptional terminator, (b) providing a carbon source and ectoine, and (c) cultivating the bacterial host cell under suitable conditions to allow biocatalytic conversion of ectoine into hydroxyectoine, and thereby secreting the produced hydroxyectoine. The invention further relates to a recombinant bacterial host cell comprising a heterologous promoter operably linked to a nucleotide sequence encoding an ectoine hydroxylase - ectD - (EC 1.14.11) followed by a transcriptional terminator; the use of the recombinant bacterial host cell of the present invention for the production of hydroxyectoine; and a kit comprising a bacterial host cell of and a further bacterial host cell of the present invention for use in a method of for producing hydroxyectoine.

### BACKGROUND OF THE INVENTION

Extremolytes enable microbes to withstand even the most extreme conditions in nature. Due to their unique protective properties, the small organic molecules, more and more, become high-value active ingredients for the cosmetics and the pharmaceutical industries. While ectoine, the industrial extremolyte flagship, has been successfully commercialized before, an economically viable route to its highly interesting derivative 5-hydroxyectoine (hydroxyectoine) is not existing.

Extremophilic organisms accumulate and secrete extremolytes, small organic metabolites that protect them at high salinity, high temperature, and in other extreme habitats (Becker J, Wittmann C: Microbial production of extremolytes - high-value active ingredients for nutrition, health care, and well-being. Current opinion in biotechnology 2020, 65:118-128. Sahle CJ, Schroer MA, Jeffries CM, Niskanen J: Hydration in aqueous solutions of ectoine and hydroxyectoine. Physical Chemistry Chemical Physics 2018, 20:27917-27923. Schwibbert K, Marin-Sanguino A, Bagyan I, Heidrich G, Lentzen G, Seitz H, Rampp M, Schuster SC, Klenk H-P, Pfeiffer F, et al: A blueprint of ectoine metabolism from the genome of the industrial producer Halomonas elongata DSM 2581 T. Environmental microbiology 2011, 13:1973-1994. Pastor JM, Salvador M, Argandoña M, Bernal V, Reina-Bueno M, Csonka LN, Iborra JL, Vargas C, Nieto JJ, Cánovas M: Ectoines in cell stress protection: Uses and biotechnological production. Biotechnology advances 2010, 28:782-801.) [1-4]. The two most prominent extremolytes are non-proteinogenic cyclic amino acids, namely ectoine (1,4,5,6-tetrahydro-2-methyl-4-pyrimidinecarboxylic acid) and its hydroxylated derivative 5-hydroxyectoine (1,4,5,6-tetrahydro-2-methyl-5-hydroxy-4-pyrimidinecarboxylic acid, named hydroxyectoine here) (Becker J, Wittmann C: Microbial production of extremolytes - high-value active ingredients for nutrition, health care, and well-being. Current opinion in biotechnology 2020, 65:118-128. Liu M, Liu H, Shi M, Jiang M, Li L, Zheng Y: Microbial production of ectoine and hydroxyectoine as high-value chemicals. Microbial Cell Factories 2021, 20:76.) [1, 5]. Both molecules provide excellent cosmotropic [1], protein- and DNA-stabilizing (Pastor JM, Salvador M, Argandoña M, Bernal V, Reina-Bueno M, Csonka LN, Iborra JL, Vargas C, Nieto JJ, Cánovas M: Ectoines in cell stress protection: Uses and biotechnological production. Biotechnology advances 2010, 28:782-801. Kuhlmann AU, Hoffmann T, Bursy J, Jebbar M, Bremer E: Ectoine and hydroxyectoine as protectants against osmotic and cold stress: uptake through the SigB-controlled betaine-choline-carnitine transporter-type carrier EctT from Virgibacillus pantothenticus. Journal of bacteriology 2011, 193:4699-4708. Smiatek J, Harishchandra RK, Rubner O, Galla H-J, Heuer A: Properties of compatible solutes in aqueous solution. Biophysical chemistry 2012, 160:62-68.) [4, 6, 7], as well as cell- and tissue-protecting and moisturizing properties (Pastor JM, Salvador M, Argandoña M, Bernal V, Reina-Bueno M, Csonka LN, Iborra JL, Vargas C, Nieto JJ, Cánovas M: Ectoines in cell stress protection: Uses and biotechnological production. Biotechnology advances 2010, 28:782-801. Kuhlmann AU, Hoffmann T, Bursy J, Jebbar M, Bremer E: Ectoine and hydroxyectoine as protectants against osmotic and cold stress: uptake through the SigB-controlled betaine-choline- carnitine transporter-type carrier EctT from Virgibacillus pantothenticus. Journal of bacteriology 2011, 193:4699-4708. Smiatek J, Harishchandra RK, Rubner O, Galla H-J, Heuer A: Properties of compatible solutes in aqueous solution. Biophysical chemistry 2012, 160:62-68. Argandoña M, Nieto JJ, Iglesias-Guerra F, Calderón MI, Garcia-Estepa R, Vargas C: Interplay between iron homeostasis and the osmotic stress response in the halophilic bacterium Chromohalobacter salexigens. Applied and Environmental Microbiology 2010, 76:3575-3589.) [4, 6-8], which easily explains why they are of increasing commercial interest. Notably, the two ectoines are applied in high-price sectors such as cosmetics (Graf R, Anzali S, Buenger J, Pfluecker F, Driller H: The multifunctional role of ectoine as a natural cell protectant. Clinics in dermatology 2008, 26:326-333. Kunte HJ, Lentzen G, Galinski E: Industrial Production of the Cell Protectant Ectoine: Protection Mechanisms, Processes, and Products. Current Biotechnology 2014, 3:10-25.) [9, 10], medicine (Jorge CD, Borges N, Bagyan I, Bilstein A, Santos H: Potential applications of stress solutes from extremophiles in protein folding diseases and healthcare. Extremophiles 2016, 20:251-259. Bownik A, Stepniewska Z: Ectoine as a promising protective agent in humans and animals. Archives of Industrial Hygiene and Toxicology 2016, 67:260-265. Eichel A, Wittig J, Shah-Hosseini K, Mösges R: A prospective, controlled study of SNS01 (ectoine nasal spray) compared to BNO-101 (phytotherapeutic dragées) in patients with acute rhinosinusitis. Current medical research and opinion 2013, 29:739-746.) [11-13] and biotechnology (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110. Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417. Pérez-García F, Ziert C, Risse JM, Wendisch VF: Improved fermentative production of the compatible solute ectoine by Corynebacterium glutamicum from glucose and alternative carbon sources. Journal of Biotechnology 2017, 258:59-68. Ning Y, Wu X, Zhang C, Xu Q, Chen N, Xie X: Pathway construction and metabolic engineering for fermentative production of ectoine in Escherichia coli. Metabolic Engineering 2016, 36:10-18.) [14-17]. Ectoine is the industrial flagship among the world's extremolytes. The compound is produced in commercial scale by the company bitop AG (Dortmund, Germany) using the natural producer Halomonas elongata under high-salinity conditions (Pastor JM, Salvador M, Argandoña M, Bernal V, Reina-Bueno M, Csonka LN, Iborra JL, Vargas C, Nieto JJ, Cánovas M: Ectoines in cell stress protection: Uses and biotechnological production. Biotechnology advances 2010, 28:782-801. Kunte HJ, Lentzen G, Galinski E: Industrial Production of the Cell Protectant Ectoine: Protection Mechanisms, Processes, and Products. Current Biotechnology 2014, 3:10-25. Lentzen G, Schwarz T: Extremolytes: natural compounds from extremophiles for versatile applications. Applied microbiology and biotechnology 2006, 72:623-634.) [4, 10, 18]. The product trades at approximately 1,000 USD kg 1. In a rapidly growing market, ectoine has found its way into a range of products for human and animal health, including lung inhalation fluids, eye drops, nose sprays, and skin care products, among others.

An economically viable route to commercial hydroxyectoine is not existing to date, resulting in enormous prices around 17,000 USD kg⁻¹ (Liu M, Liu H, Shi M, Jiang M, Li L, Zheng Y: Microbial production of ectoine and hydroxyectoine as high-value chemicals. Microbial Cell Factories 2021, 20:76. Czech L, Hermann L, Stöveken N, Richter AA, Höppner A, Smits SHJ, Heider J, Bremer E: Role of the Extremolytes Ectoine and Hydroxyectoine as Stress Protectants and Nutrients: Genetics, Phylogenomics, Biochemistry, and Structural Analysis. Genes 2018, 9:177.) [5, 19]. The difficulty to produce hydroxyectoine, simply, is the unfavorable layout of the underlying biosynthetic pathway. Inevitably, hydroxyectoine is only accessible through the L-lysine pathway via ectoine as a central precursor (Czech L, Hermann L, Stöveken N, Richter AA, Höppner A, Smits SHJ, Heider J, Bremer E: Role of the Extremolytes Ectoine and Hydroxyectoine as Stress Protectants and Nutrients: Genetics, Phylogenomics, Biochemistry, and Structural Analysis. Genes 2018, 9:177.) [19] which results in the accumulation of mixtures of the two compounds, typically with larger shares of ectoine (Sauer T, Galinski EA: Bacterial milking: A novel bioprocess for production of compatible solutes. Biotechnol Bioeng 1998, 57:306-313.) [20]. Although promising attempts demonstrated *de novo* hydroxyectoine synthesis, titers have remained quite low: 0.4 g L⁻¹ in recombinant *C*. *glutamicum* (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.) [14], 2.9 g L⁻¹ in the natural producer *Halomonas salina* (Chen W-C, Hsu C-C, Wang L-F, Lan JC-W, Chang Y-K, Wei Y-H: Exploring useful fermentation strategies for the production of hydroxyectoine with a halophilic strain, Halomonas salina BCRC 17875. Journal of bioscience and bioengineering 2019, 128:332-336.) [21], and 14.9 g L⁻¹ in a recombinant *Escherichia coli* strain that required a quite complex fermentation process (Ma Q, Xia L, Wu H, Zhuo M, Yang M, Zhang Y, Tan M, Zhao K, Sun Q, Xu Q, et al: Metabolic engineering of Escherichia coli for efficient osmotic stress-free production of compatible solute hydroxyectoine. Biotechnology and bioengineering 2022, 119:89-101.) [22]. The low titers make fermentation and downstream processing too costly. An alternative approach, previously suggested to derive hydroxyectoine, is based on bioconversion from ectoine through hydroxylation (Czech L, Stöveken N, Bremer E: EctD-mediated biotransformation of the chemical chaperone ectoine into hydroxyectoine and its mechanosensitive channel-independent excretion. Microbial Cell Factories 2016, 15:126.) [23]. Heterologous expression of ectoine hydroxylase in *E*. *coli* enabled the conversion of an admittedly low amount of 2.1 g L⁻¹ ectoine into hydroxyectoine within 24 h. Clearly, hydroxyectoine production has not advanced beyond an initial proof-of-principle stage. A further example of such an initial proof-of-principle stage is disclosed in the dissertation of Jarryd Finn Brauner, 2021. Therein the hydroxylation of ectoine and synthetic ectoine derivatives via *E*. *coli* mediated biotransformation is described. The biotransformation in E. coli is conducted with the enzyme ectoine hydroxylase ectD derived from different bacteria, namely *Halomonas elongata, S. alaskensis, A*. *cryptum,* and *P*. *stutzeri.* However, according to the work of Brauner, the ectD of *P*. *stutzeri* is less effective as shown in lower in the conversion compared to the other ectD enzymes. In view of the work of Brauner, this may be reasoned in lesser enzyme stability of the ectD of *P. stutzeri.*

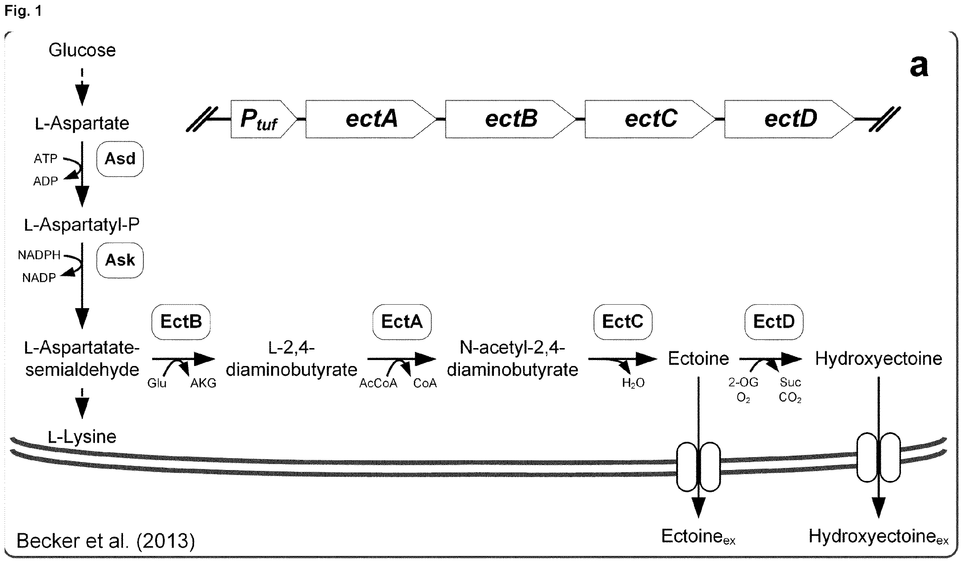

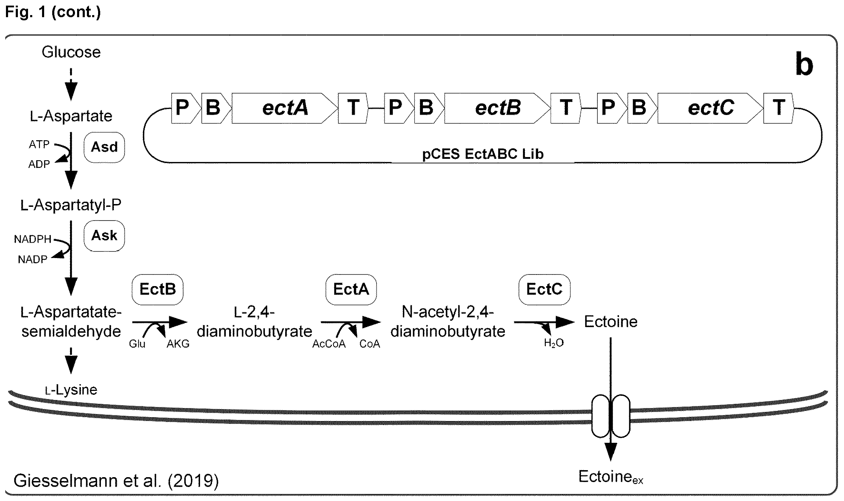

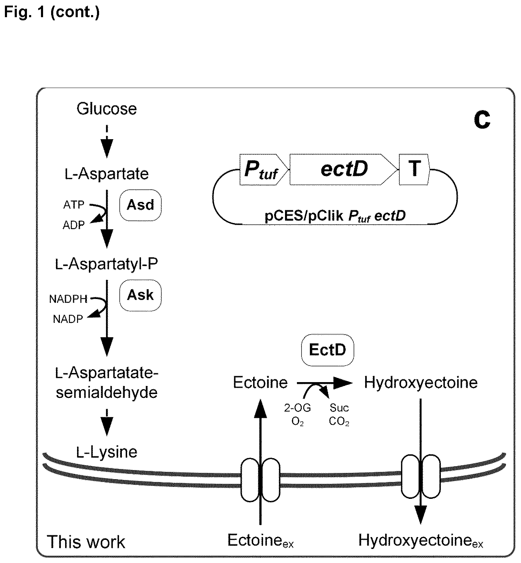

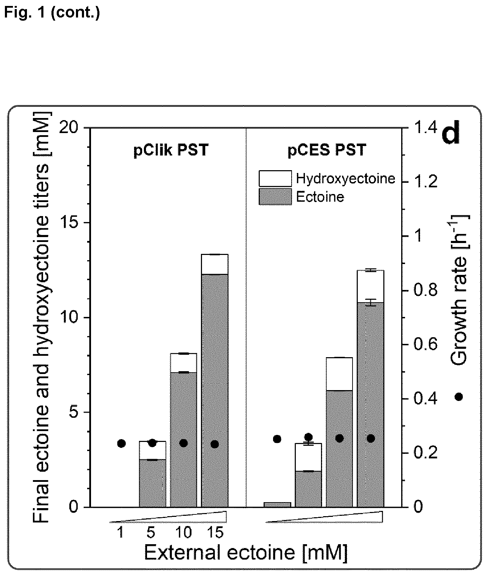

Previously, the inventors have metabolically engineered the soil microbe *C*. *glutamicum* to overproduce different L lysine-based chemicals, including L lysine itself (Hoffmann SL, Kohlstedt M, Jungmann L, Hutter M, Poblete-Castro I, Becker J, Wittmann C: Cascaded valorization of brown seaweed to produce I-lysine and value-added products using Corynebacterium glutamicum streamlined by systems metabolic engineering. Metabolic Engineering 2021, 67:293-307.) [24], diaminopentane (Kind S, Neubauer S, Becker J, Yamamoto M, Völkert M, Abendroth Gv, Zelder O, Wittmann C: From zero to hero - Production of bio-based nylon from renewable resources using engineered Corynebacterium glutamicum. Metabolic Engineering 2014, 25:113-123.) [25], glutarate (Rohles CM, Gläser L, Kohlstedt M, Gießelmann G, Pearson S, del Campo A, Becker J, Wittmann C: A bio-based route to the carbon-5 chemical glutaric acid and to bionylon-6,5 using metabolically engineered Corynebacterium glutamicum. Green Chemistry 2018, 20:4662-4674.) [26], 5-aminovalerate (Rohles CM, Pauli S, Giesselmann G, Kohlstedt M, Becker J, Wittmann C: Systems metabolic engineering of Corynebacterium glutamicum eliminates all by-products for selective and high-yield production of the platform chemical 5-aminovalerate. Metabolic Engineering 2022, 73:168-181.) [27], and, last but not least, mixtures of ectoine and hydroxyectoine (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.) [14] as well as pure ectoine (Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.) [15]. The latter two studies provided valuable proof-of-concept that *C*. *glutamicum* expresses heterologous proteins that convert L-aspartate semialdehyde, an intermediate of L-lysine biosynthesis (Wittmann C, Heinzle E: Modeling and experimental design for metabolic flux analysis of lysine-producing corynebacteria by mass spectrometry. Metab Eng 2001, 3:173-191.) [28], into the two ectoines (Fig. 1 a,b).

In view of the above-described limitations of the production of hydroxyectoine in the prior art, there is a need for the provision of a method and means which allow a more efficient production of hydroxyectoine.

### SUMMARY OF THE INVENTION

The present invention means and methods which allow the advantageous production of hydroxyectoine in a larger scale which is more efficient and less time and cost intensive. In particular the present invention provides in a first aspect a method for producing hydroxyectoine, comprising (a) providing a biocatalyst, wherein said biocatalyst is a recombinant bacterial host cell transformed with a promoter operably linked to a nucleotide sequence encoding an ectoine hydroxylase - ectD - (EC 1.14.11) followed by a transcriptional terminator, (b) providing a carbon source and ectoine, and (c) cultivating the bacterial host cell under suitable conditions to allow biocatalytic conversion of ectoine into hydroxyectoine, and thereby secreting the produced hydroxyectoine.

The present invention provides in a second aspect a recombinant bacterial host cell comprising a promoter operably linked to a nucleotide sequence encoding an ectoine hydroxylase - ectD - (EC 1.14.11) followed by a transcriptional terminator.

In a third aspect, the present invention is directed to the use of the recombinant bacterial host cell of the invention for the production of hydroxyectoine.

In a fourth aspect, the present invention is directed to a kit comprising a bacterial host cell of the invention and a further bacterial host cell of the invention for use in a method of the invention.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

### Overview of the sequence listing

| **SEQ ID NO:** | |
|---|---|
| 1 | pCES_PLPV empty vector |
| 2 | pClik_5α_MCS empty vector |
| 3 | Ptuf |
| 4 | ectA_Pseudomonas_stutzeri_co |
| 5 | ectB_Pseudomonas_stutzeri_co |
| 6 | ectC_Pseudomonas_stutzeri_co |
| 7 | Pseudomonas stutzeri A1501 ectD - original |
| 8 | Pseudomonas stutzeri A1501 ectD - optimized |
| 9 | Mycobacterium smegmatis ATCC 19420 ectD - original |
| 10 | Mycobacterium smegmatis ATCC 19420 ectD - optimized |
| 11 | Streptomyces coelicolor A3(2) ectD - original |
| 12 | Streptomyces coelicolor A3(2) ectD - optimized |
| 13 | Halomonas elongata ATCC 33173 ectD - original |
| 14 | Halomonas elongata ATCC 33173 ectD - optimized |
| 15 | Virgibacillus salexigens ATCC 700290 ectD - original |
| 16 | Virgibacillus salexigens ATCC 700290 ectD - optimized |
| 17 | Gracilibacillus sp. SCU50 ectD - original |
| 18 | Gracilibacillus sp. SCU50 ectD - optimized |
| 19 | Acidiphilium cryptum JF5 ectD - original |
| 20 | Acidiphilium cryptum JF5 ectD - optimized |
| 21 | Alkalihalobacillus clausii 7520-2 ectD - original |
| 22 | Alkalihalobacillus clausii 7520-2 ectD - optimized |
| 23 | Hydrocarboniclastica marina KCTC 62334 ectD - original |
| 24 | Hydrocarboniclastica marina KCTC 62334 ectD - optimized |
| 25 | Methylomicrobium alcaliphilum DSM 19304 ectD - original |
| 26 | Methylomicrobium alcaliphilum DSM 19304 ectD - optimized |
| 27 | Neptunomonas concharum LHW37 ectD - original |
| 28 | Neptunomonas concharum LHW37 ectD - optimized |
| 29 | Leptospirillum ferriphilum ML-04 ectD - original |
| 30 | Leptospirillum ferriphilum ML-04 ectD - optimized |
| 31 | Candidatus Nitrosopumilus sp. AR2 ectD - original |
| 32 | Candidatus Nitrosopumilus sp. AR2 ectD - optimized |
| 33 | Sphyngopyxis alaskensis DSM 13593 ectD - original |
| 34 | Sphyngopyxis alaskensis DSM 13593 ectD - optimized |
| 35 | Paenibacillus lautus E7593-69 ectD - original |
| 36 | Paenibacillus lautus E7593-69 ectD - optimized |
| 37 | Chromohalobacter salexigens DSM 3043 ectD - original |
| 38 | Chromohalobacter salexigens DSM 3043 ectD - optimized |
| 39 | pCES MSM Ptuf fw |
| 40 | pCES MSM Ptuf rv |
| 41 | pCES MSM ectD fw |
| 42 | pCES MSM ectD rv |
| 43 | pCES VSA Ptuf fw |
| 44 | pCES VSA Ptuf rv |
| 45 | pCES VSA ectD fw |
| 46 | pCES VSA ectD rv |
| 47 | pCES SCO Ptuf fw |
| 48 | pCES SCO Ptuf rv |
| 49 | pCES SCO ectD fw |
| 50 | pCES SCO ectD rv |
| 51 | pCES PST Ptuf fw |
| 52 | pCES PST Ptuf rv |
| 53 | pCES PST ectD fw |
| 54 | pCES PST ectD rv |
| 55 | pCES HEL Ptuf fw |
| 56 | pCES HEL Ptuf rv |
| 57 | pCES HEL ectD fw |
| 58 | pCES HEL ectD rv |
| 59 | pClik MSM Ptuf fw |
| 60 | pClik MSM Ptuf rv |
| 61 | pClik MSM ectD fw |
| 62 | pClik MSM ectD rv |
| 63 | pClik VSA Ptuf fw |
| 64 | pClik VSA Ptuf rv |
| 65 | pClik VSA ectD fw |
| 66 | pClik VSA ectD rv |
| 67 | pClik SCO Ptuf fw |
| 68 | pClik SCO Ptuf rv |
| 69 | pClik SCO ectD fw |
| 70 | pClik SCO ectD rv |
| 71 | pClik PST Ptuf fw |
| 72 | pClik PST Ptuf rv |
| 73 | pClik PST ectD fw |
| 74 | pClik PST ectD rv |
| 75 | pClik HEL Ptuf fw |
| 76 | pClik HEL Ptuf rv |
| 77 | pClik HEL ectD fw |
| 78 | pClik HEL ectD rv |

### BRIEF DESCRIPTION OF THE FIGURES

**Figure 1****:** As part of the L-lysine biosynthetic pathway, L-aspartate is converted into L-aspartate-semialdehyde, involving aspartokinase (Ask; EC: 2.7.2.4) and L-aspartyl phosphate dehydrogenase (Asd; EC: 1.2.1.11). Synthesis of ectoines branches off from the L-aspartate-semialdehyde pool. First, L-2,4-diaminobutyrate transaminase (EctB; EC: 2.6.1.76) catalyzes the transamination to L-2,4-diaminobutyrate. Then, L-2,4-diaminobutyrate acetyltransferase (EctA; EC: 2.3.1.178) acetylates the intermediate to N-acetyl-2,4-diaminobutyrate. Finally, ectoine is cyclized by ectoine synthase (EctC; EC: 4.2.1.108). A fourth enzyme, i. e. ectoine hydroxylase (EctD; EC: 1.14.11), further converts ectoine into 5-hydroxyectoine *via* O₂-dependent hydroxylation, simultaneously decarboxylating the co-substrate α-ketoglutarate. Previously, overproduction of a mixture of ectoine and 5-hydroxyectoine in engineered *C*. *glutamicum* ECT-2 (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.) [14] was achieved by genomic integration of the (codon-optimized) *ectABCD* cluster from *Pseudomonas stutzeri* under control of the promoter *P_{tuf}* (a). Pure ectoine production in C. *glutamicum ectABC^{opt}* (Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.) [15] was based on episomal monocistronic expression of the codon-optimized *ect^{ABC}* genes from *P. stutzeri,* each under control of a distinct promoter (P), a bicistronic element (B) and a terminator sequence (T), respectively (b). Metabolic engineering strategy of this work to convert ectoine to hydroxyectoine in a biotransformation set-up using recombinant *C*. *glutamicum* that expresses *ectD* under control of *P_{tuf}* (c). Experimental proof of hydroxyectoine production using the biotransformation set-up. The *C. glutamicum* type strain ATCC 13032 episomally expressed the codon optimized *ectD* gene from *Pseudomonas stutzeri* A1501 (PST) in the pClik 5a (pClik) and pCES-PLPV (pCES) vector. Cultures were grown at 30°C on minimal glucose medium in a microbioreactor with different initial levels of supplied ectoine (1, 5, 10, 15 mM) and analyzed for growth (on-line measurement of OD₆₂₀) and the conversion of ectoine into 5-hydroxyectoine (final titers after depletion of glucose) (d). n=2.

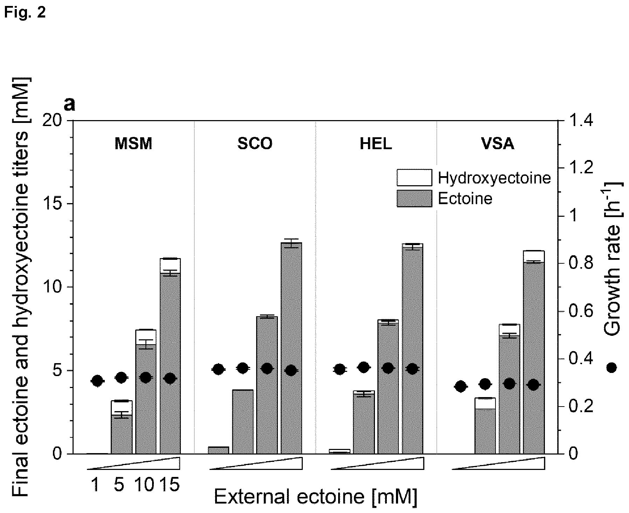

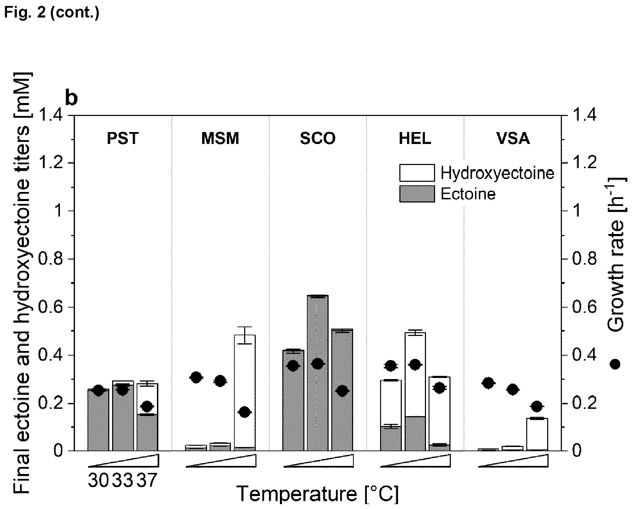

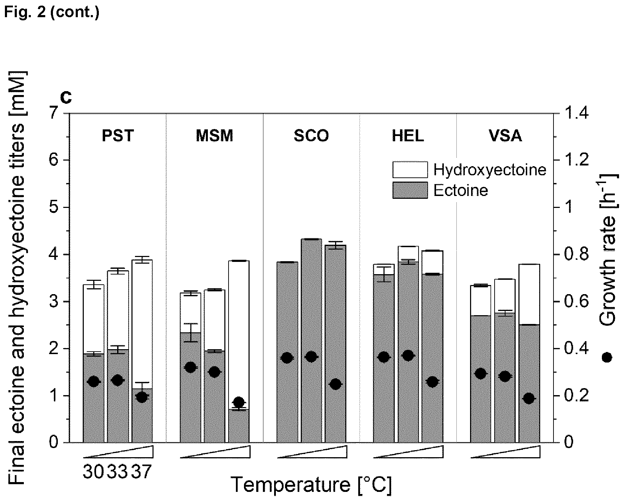

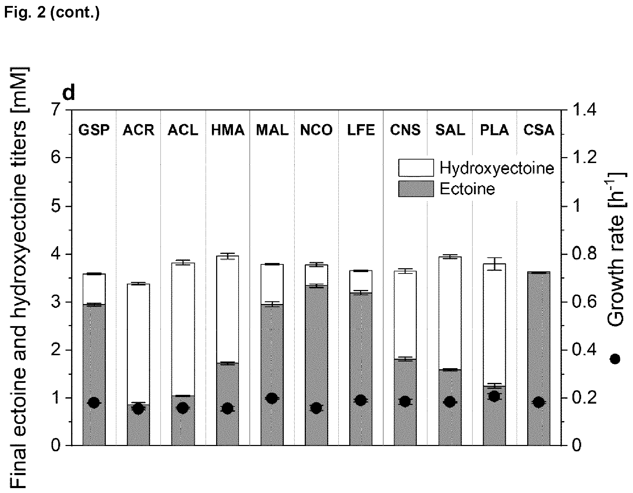

**Figure 2****:** Screening for optimal 5-hydroxyectoine production in recombinant *C*. *glutamicum.* The *C*. *glutamicum* type strain ATCC 13032 episomally expressed the codon optimized *ectD* genes from *Pseudomonas stutzeri* A1501 (PST), *Mycobacterium smegmatis* ATCC 19420 (MSM), *Streptomyces coelicolor* A3(2) (SCO), *Halomonas elongata* ATCC 33173 (HEL) and *Virgibacillus salexigens* ATCC 700290 with amino acid exchanges A163C and S244C (VSA), in the pCES-PLPV vector. Cultures were grown at 30°C on minimal glucose medium in a microbioreactor and analyzed for growth (on-line measurement of OD₆₂₀) and the conversion of ectoine into hydroxyectoine (final titers after depletion of glucose). Screening at different initial ectoine concentrations (a), and different temperatures (b, c). n=2. Screening of additional EctD enzyme variants in *C*. *glutamicum* at 37°C and 5 mM ectoine (d). For the latter, *C*. *glutamicum* episomally expressed the codon optimized *ectD* genes from *Gracilibacillus sp.* SCU50 (GSP), *Acidiphilium cryptum* JF5 (ACR), *Alkalihalobacillus clausii* 7520-2 (ACL), *Hydrocarboniclastica marina* KCTC 62334 (HMA), *Methylomicrobium alcaliphilum* DSM 19304 (MAL), *Neptunomonas concharum* LHW37 (NCO), *Leptospirillum ferriphilum* ML-04 (LFE), *Candidatus Nitrosopumilus* sp. AR2 (CNS), *Sphingopyxis alaskensis* DSM 13593 (SAL), *Paenibacillus lautus* E7593-69 (PLA) and *Chromohalobacter salexigens* DSM 3043 (CSA), in the pCES-PLPV vector. The cultures were grown at 37°C on minimal glucose medium in a microbioreactor and analyzed for growth (on-line measurement of OD₆₂₀) and the conversion of 5 mM initial ectoine into hydroxyectoine (final titers after depletion of glucose). n=3.

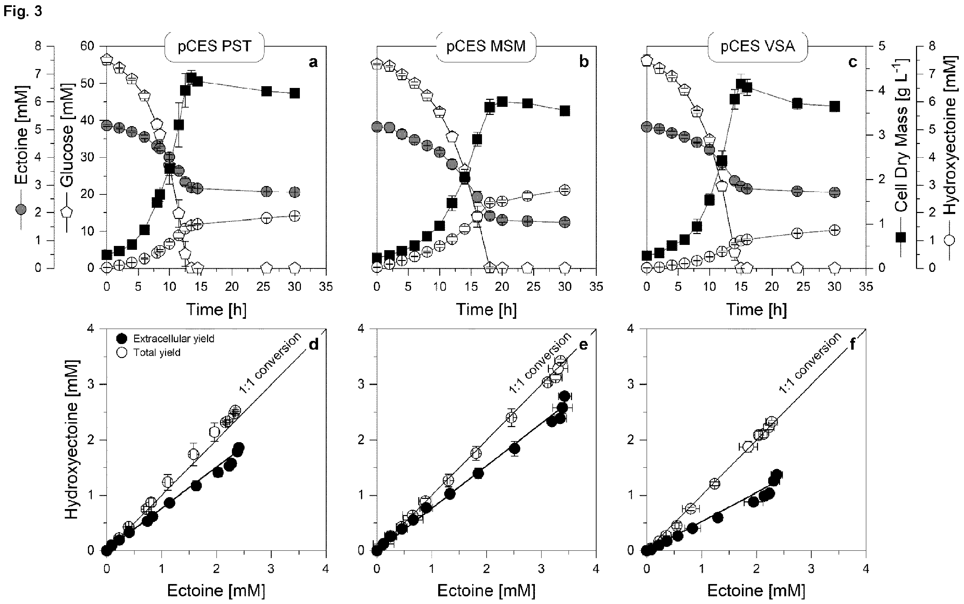

**Figure 3****:** Kinetics and stoichiometry of 5-hydroxyectoine production in metabolically engineered strains of *C*. *glutamicum.* The *C*. *glutamicum* type strain ATCC 13032 episomally expressed the codon optimized *ectD* genes from *Pseudomonas stutzeri* A1501 (a, c), *Mycobacterium smegmatis* ATCC 19420 (b, e). and *Virgibacillus salexigens* ATCC 700290 with amino acid exchanges A163C and S244C (c, f) in the pCES-PLPV vector. All strains were cultivated in shake flasks on glucose minimal medium with 5 mM of ectoine at 37°C. Dark grey circles show conversion based on extracellularly detectable ectoines. Light grey circles indicate conversion additionally considering intracellular levels of ectoines. n=3.

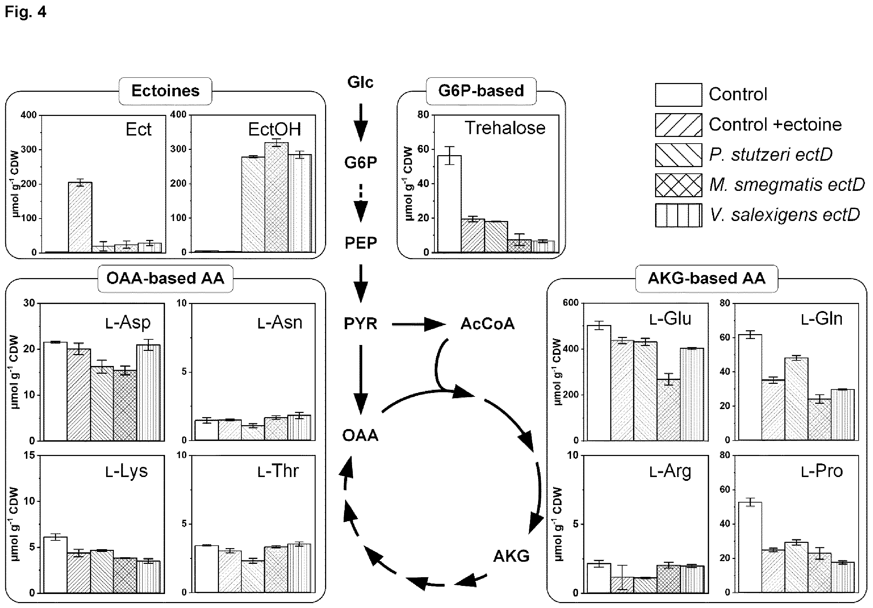

**Figure 4****:** Impact of ectoine and 5-hydroxyectoine on intracellular metabolite levels in *C*. *glutamicum.* The *C*. *glutamicum* type strain ATCC 13032, episomally expressed the codon-optimized *ectD* genes from *Pseudomonas stutzeri* A1501, *Mycobacterium smegmatis* ATCC 19420 and *Virgibacillus salexigens* ATCC 700290 with amino acid exchanges A163C and S244C in the pCES-PLPV vector. All strains were cultivated in shake flasks on glucose minimal medium supplemented with 5 mM ectoine at 37°C. Cells were harvested in mid-exponential phase (10 h) and disrupted for the analysis of intracellular metabolite levels. As a control, C. *glutamicum* harboring the empty vector pCES-PLPV was treated equally with (Control +ectoine) and without (Control) the addition of ectoine to the medium. n=3.

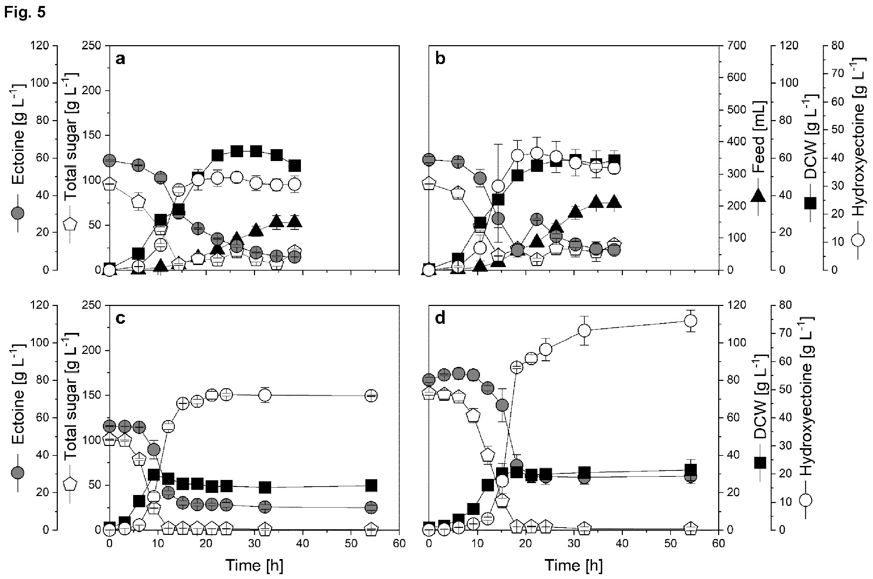

**Figure 5****:** Production of 5-hydroxyectoine in stirred tank bioreactors using metabolically engineered *C*. *glutamicum.* The *C*. *glutamicum* type strain ATCC 13032 episomally expressed the codon optimized *ectD* genes from *Mycobacterium smegmatis* ATCC 19420 (a) and *Pseudomonas stutzeri* A1501 (b-d). Fed-batch-mode process at 37°C with initial sucrose and ectoine concentrations of 100 g L⁻¹ and 60 g L⁻¹, and the addition of feed (600 g L⁻¹ sucrose, 15 g L⁻¹ yeast extract) at constant rate, respectively (a, b). Batch-mode process at 37°C with initial sucrose and ectoine concentrations of 100 g L⁻¹ and 50 g L⁻¹ (c) and 150 g L⁻¹ and 75 g L⁻¹ (d). n=2.

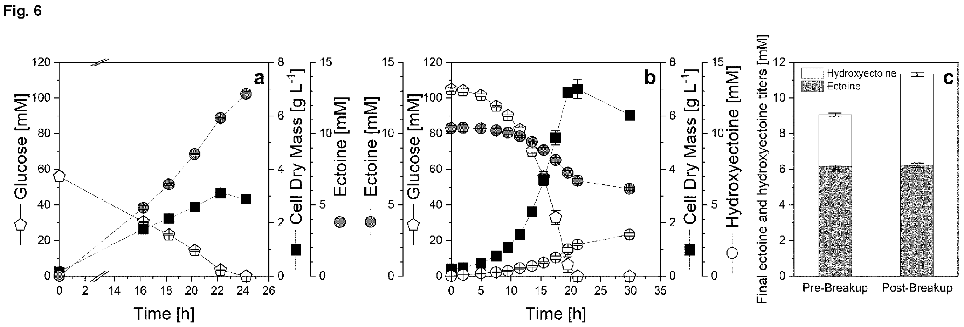

**Figure 6****:** *De-novo* synthesis of hydroxyectoine using a two-step bioprocess. Step one: cultivation of the ectoine producer *C*. *glutamicum ectABC^{opt}* (Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.) [15] on 10 g L⁻¹ glucose minimal medium at 30°C (a). Step two: cultivation of C. *glutamicum* episomally expressing the codon optimized *ectD* gene from *Mycobacterium smegmatis* ATCC 19420 at 37°C. The medium displayed the final broth from step 1, clarified from the ectoine-producing cells, neutralized to pH 7.4, and replenished with glucose (20 g L⁻¹) (b). Final ectoine and hydroxyectoine titers of phase 2 before and after cell disruption (c). n=3.

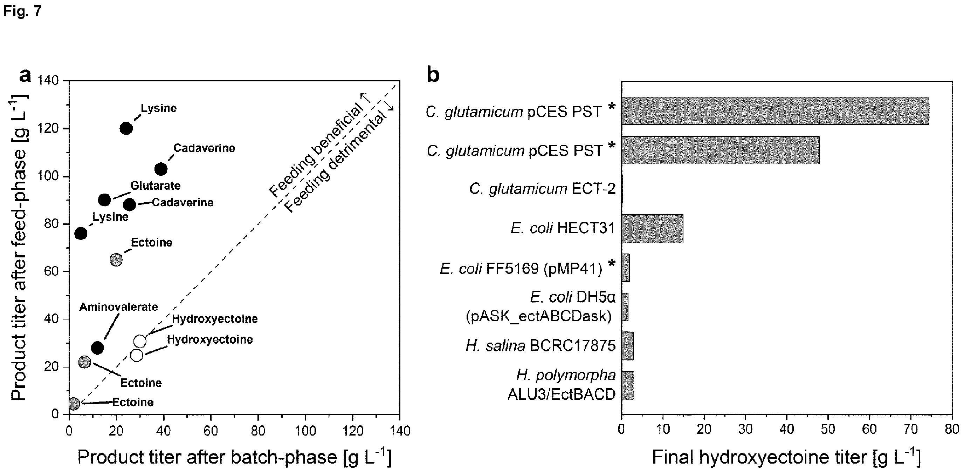

**Figure 7****:** Benchmarking the newly developed 5-hydroxyectoine production process based on metabolically engineered *C*. *glutamicum.* Comparison of fed-batch processes using *C*. *glutamicum* to produce I-aspartate derived chemicals. Product titers, achieved after the initial batch-phase, are plotted against final titers, reached after the feed-phase. Blue circles: *de novo* production of I-lysine and products derived thereof (Hoffmann SL, Kohlstedt M, Jungmann L, Hutter M, Poblete-Castro I, Becker J, Wittmann C: Cascaded valorization of brown seaweed to produce I-lysine and value-added products using Corynebacterium glutamicum streamlined by systems metabolic engineering. Metabolic Engineering 2021, 67:293-307. Kind S, Neubauer S, Becker J, Yamamoto M, Völkert M, Abendroth Gv, Zelder O, Wittmann C: From zero to hero - Production of bio-based nylon from renewable resources using engineered Corynebacterium glutamicum. Metabolic Engineering 2014, 25:113-123. Rohles CM, Gläser L, Kohlstedt M, Gießelmann G, Pearson S, del Campo A, Becker J, Wittmann C: A bio-based route to the carbon-5 chemical glutaric acid and to bionylon-6,5 using metabolically engineered Corynebacterium glutamicum. Green Chemistry 2018, 20:4662-4674. Becker J, Zelder O, Häfner S, Schröder H, Wittmann C: From zero to hero-Design-based systems metabolic engineering of Corynebacterium glutamicum for I-lysine production. Metabolic Engineering 2011, 13:159-168. Rohles CM, Gießelmann G, Kohlstedt M, Wittmann C, Becker J: Systems metabolic engineering of Corynebacterium glutamicum for the production of the carbon-5 platform chemicals 5-aminovalerate and glutarate. Microbial Cell Factories 2016, 15:154. Buschke N, Becker J, Schäfer R, Kiefer P, Biedendieck R, Wittmann C: Systems metabolic engineering of xylose-utilizing Corynebacterium glutamicum for production of 1,5-diaminopentane. Biotechnology journal 2013, 8:557-570.) [24-26, 30, 48, 851; light orange circles: *de novo* synthesis of ectoine (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110. Pérez-García F, Ziert C, Risse JM, Wendisch VF: Improved fermentative production of the compatible solute ectoine by Corynebacterium glutamicum from glucose and alternative carbon sources. Journal of Biotechnology 2017, 258:59-68.) [14, 16]; dark orange circles: bioconversion of extracellularly provided ectoine (this work) (a). Comparison of 5-hydroxyectoine titers of various microbial producers, namely *C. glutamicum P_{tuf} ectD^{PST}* in a 1.5-fold (74.4 g L⁻¹) and a 1-fold batch (47.8 g L⁻¹) (this work), *C. glutamicum* ECT-2 (0.3 g L⁻¹) (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110. ) [14], *E. coli* HECT31 (14.9 g L⁻¹) (Ma Q, Xia L, Wu H, Zhuo M, Yang M, Zhang Y, Tan M, Zhao K, Sun Q, Xu Q, et al: Metabolic engineering of Escherichia coli for efficient osmotic stress-free production of compatible solute hydroxyectoine. Biotechnology and bioengineering 2022, 119:89-101.) [22], *E. coli* FF5169 pMP41 (1.9 g L⁻¹) (Czech L, Stöveken N, Bremer E: EctD-mediated biotransformation of the chemical chaperone ectoine into hydroxyectoine and its mechanosensitive channel-independent excretion. Microbial Cell Factories 2016, 15:126.) [23], *E. coli* DH5α pASK *ectABCDask* (1.6 g L⁻¹) (Bethlehem L, Moritz KD: Boosting Escherichia coli's heterologous production rate of ectoines by exploiting the non-halophilic gene cluster from Acidiphilium cryptum. Extremophiles 2020, 24:733-747.) [86], *H. salina* BCRC17875 (2.9 g L⁻¹) (Chen W-C, Hsu C-C, Wang L-F, Lan JC-W, Chang Y-K, Wei Y-H: Exploring useful fermentation strategies for the production of hydroxyectoine with a halophilic strain, Halomonas salina BCRC 17875. Journal of bioscience and bioengineering 2019, 128:332-336.) [21] and *H. polymorpha* ALU3/EctABCD (2.8 g L⁻¹) (Eilert E, Kranz A, Hollenberg CP, Piontek M, Suckow M: Synthesis and release of the bacterial compatible solute 5-hydroxyectoine in Hansenula polymorpha. Journal of Biotechnology 2013, 167:85-93.) [87]. Bioconversions are marked with stars.

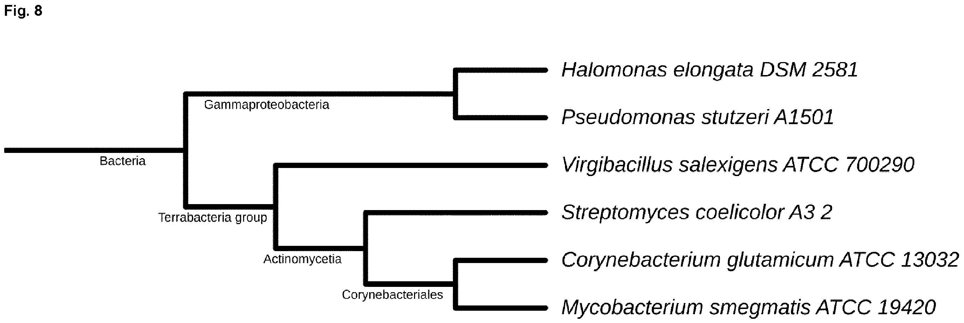

**Figure 8****:** Phylogenetic analysis of the donor organisms used in this work. The phylogenetic tree of the donor organisms and the host *C. glutamicum* ATCC 13032 was compiled using the phyloT online tool (biobyte solutions GmbH, Heidelberg, Germany) and trees visualized with the iTOL online tool (Letunic I, Bork P: Interactive Tree Of Life (iTOL) v5: an online tool for phylogenetic tree display and annotation. Nucleic Acids Res 2021, 49:W293-W296.) [1].

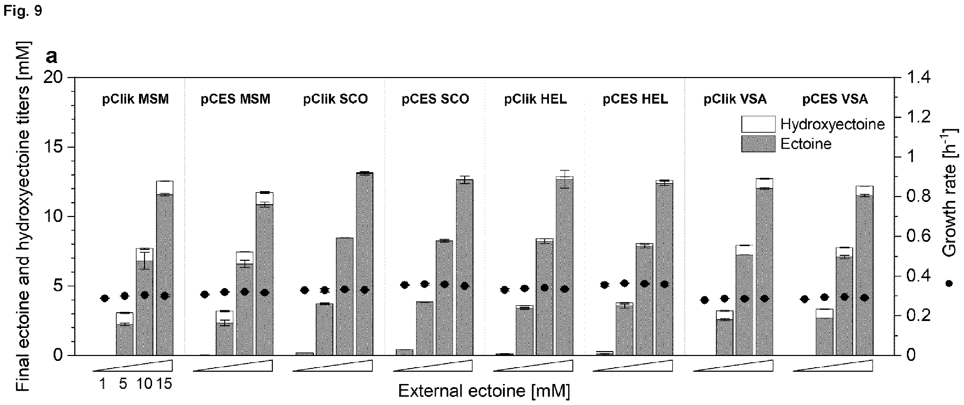

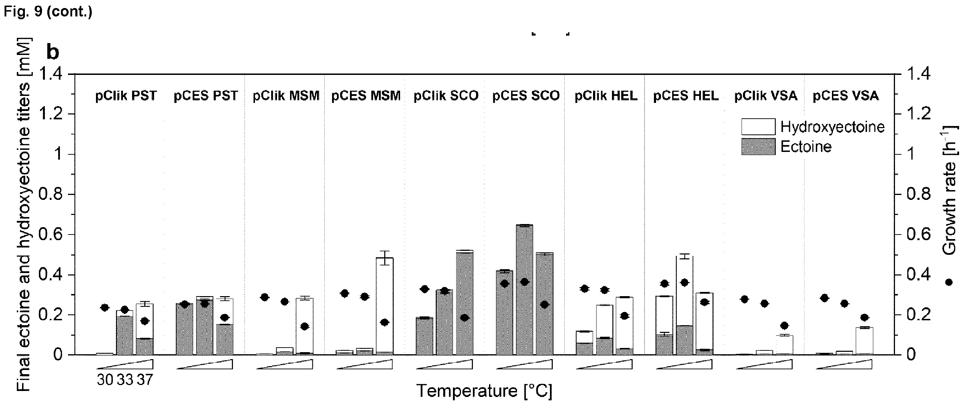

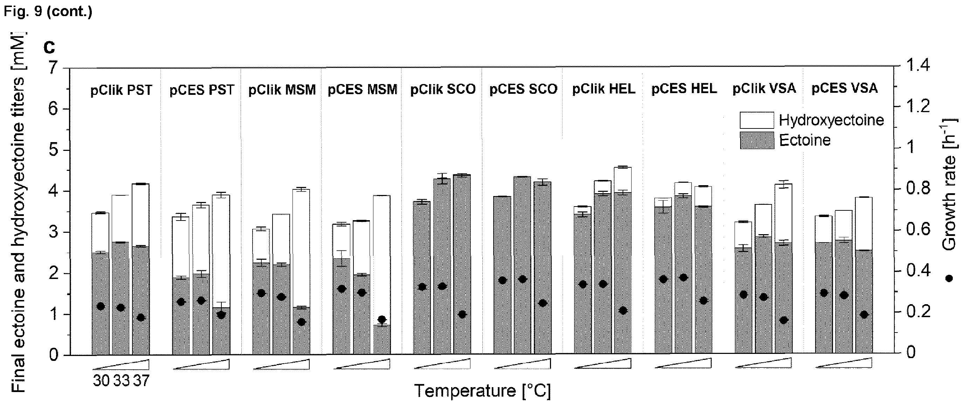

**Figure 9****:** Screening for optimal 5-hydroxyectoine production in recombinant *C*. *glutamicum.* The *C*. *glutamicum* type strain ATCC 13032 episomally expressed the codon optimized *ectD* genes from *Pseudomonas stutzeri* A1501 (PST), *Mycobacterium smegmatis* ATCC 19420 (MSM), *Streptomyces coelicolor* A3(2) (SCO), *Halomonas elongata* ATCC 33173 (HEL) and *Virgibacillus salexigens* ATCC 700290 with amino acid exchanges A163C and S244C (VSA) (Widderich N, Pittelkow M, Höppner A, Mulnaes D, Buckel W, Gohlke H, Smits SHJ, Bremer E: Molecular Dynamics Simulations and Structure-Guided Mutagenesis Provide Insight into the Architecture of the Catalytic Core of the Ectoine Hydroxylase. J Mol Biol 2014, 426:586-600.) [2], in the pClik 5a (pClik) and pCES-PLPV (pCES) vector. Cultures were grown on minimal glucose medium in a microbioreactor and analyzed for growth (on-line measurement of OD₆₂₀) and the conversion of ectoine into hydroxyectoine (final titers after depletion of glucose). Screening for different initial ectoine concentrations at 30°C (a). Screening at different temperatures with 1 mM and 5 mM initial ectoine, respectively (b, c). n=2.

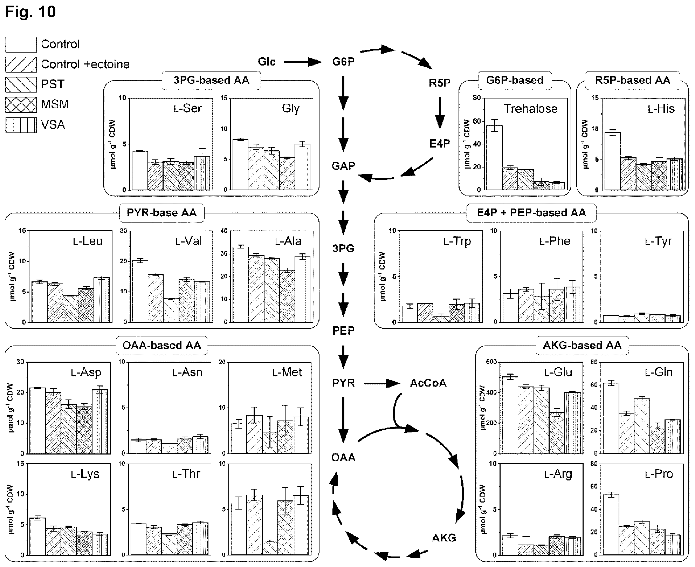

**Figure 10****:** Impact of ectoine and 5-hydroxyectoine on intracellular metabolite levels in C. *glutamicum.* For 5-hydroxyectoine production *C*. *glutamicum* ATCC 13032, episomally expressed a codon-optimized *ectD* gene from *Pseudomonas stutzeri* A1501 (PST), *Mycobacterium smegmatis* ATCC 19420 (MSM), or *Virgibacillus salexigens* ATCC 700290 (VSA), respectively. The gene from *V. salexigens* encoded for an enzyme variant that carried the amino acid exchanges A163C and S244C (Widderich N, Pittelkow M, Höppner A, Mulnaes D, Buckel W, Gohlke H, Smits SHJ, Bremer E: Molecular Dynamics Simulations and Structure-Guided Mutagenesis Provide Insight into the Architecture of the Catalytic Core of the Ectoine Hydroxylase. J Mol Biol 2014, 426:586-600.) [2]. All strains were cultivated at 37°C on minimal glucose medium, supplemented with 5 mM ectoine. Cells were analyzed for intracellular metabolite levels during the mid-exponential phase (10 h). As a control, *C. glutamicum,* harboring the empty vector, investigated during growth on glucose and ectoine (Control) and during growth on glucose alone (Control -Ect), respectively. n=3.

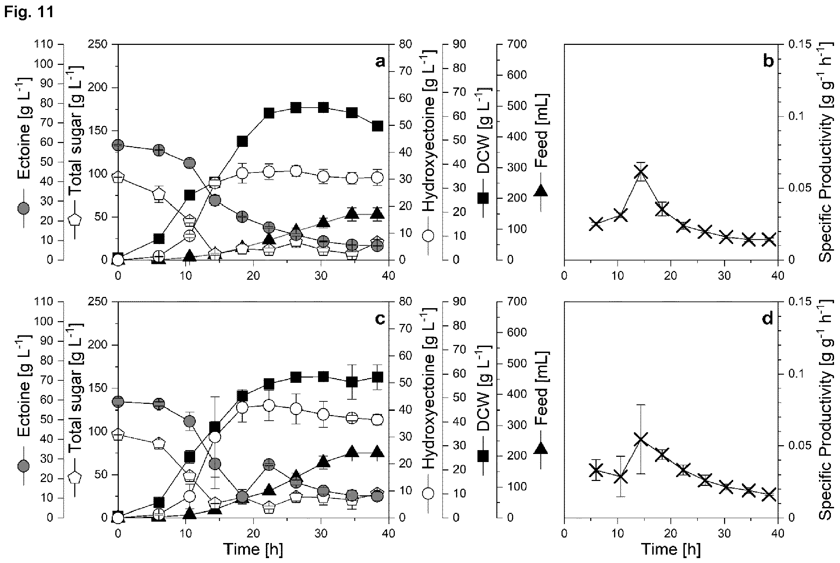

**Figure 11****:** Production of 5-hydroxyectoine in a fed-batch process in stirred tank bioreactors using metabolically engineered *C*. *glutamicum.* The *C*. *glutamicum* type strain ATCC 13032 episomally expressed the codon optimized *ectD* genes from *Mycobacterium smegmatis* ATCC 19420 (a, b) and *Pseudomonas stutzeri* A1501 (c, d). The process was operated in fed-batch-mode at 37°C with initial sucrose and ectoine concentrations of 100 g L⁻¹ and 60 g L⁻¹, and the addition of feed (600 g L⁻¹ sucrose, 15 g L⁻¹ yeast extract) at constant rate, respectively. n=2.

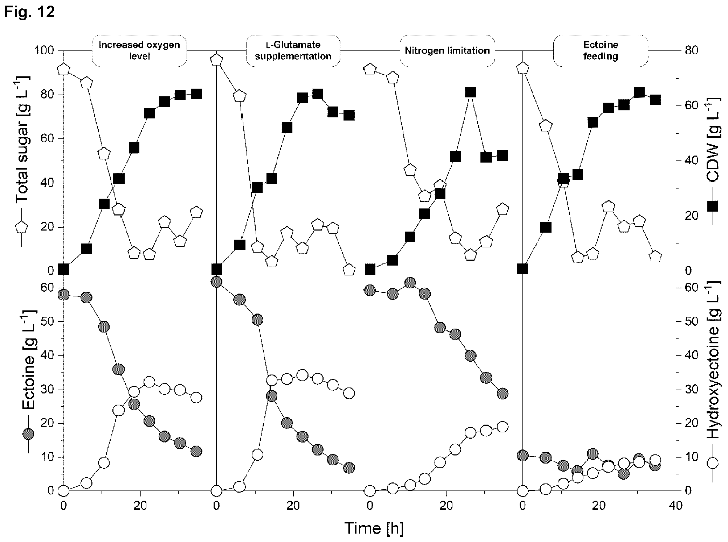

**Figure 12****:** Impact of process conditions on fed-batch production of 5-hydroxyectoine using strain *C*. *glutamicum P_{tuf} ectD^{MSM}* in a stirred tank bioreactor at 37°C. In each setup one process parameter was varied as compared to the standard layout (Fig. 7) to study its impact (from left to right): DO maintained at 60%, twice as high as in the control; supplementation of batch and feed medium with I-glutamate; limited nitrogen supply by elimination of small amounts of yeast extract from the batch medium and the use of a nitrogen-free feed; addition of ectoine at a reduced initial amount (10 g L⁻¹) plus separate feeding later on. n=1.

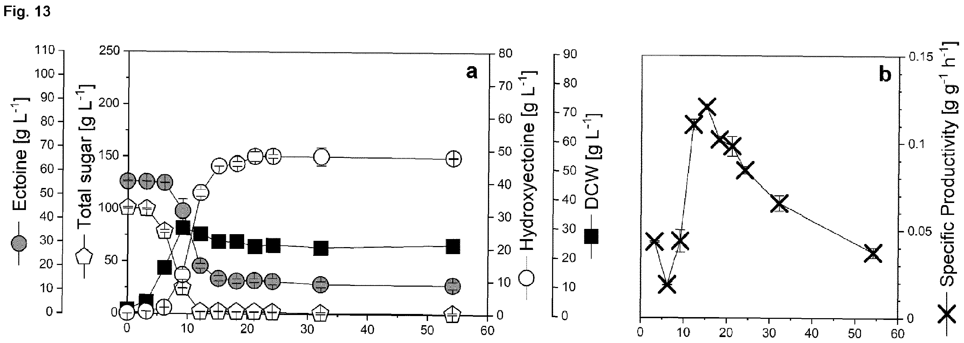

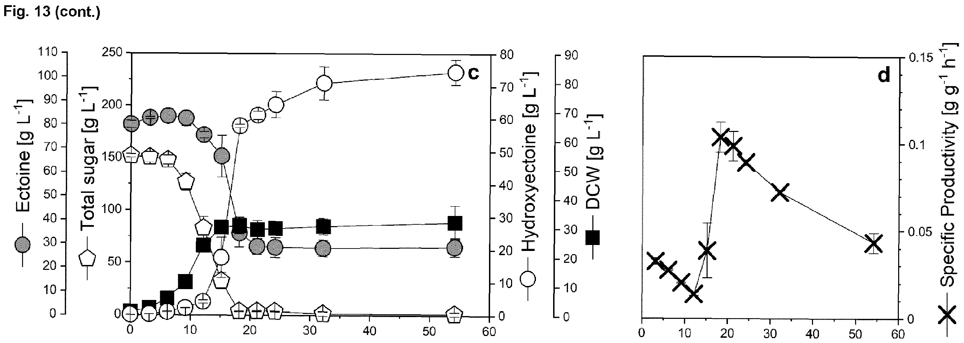

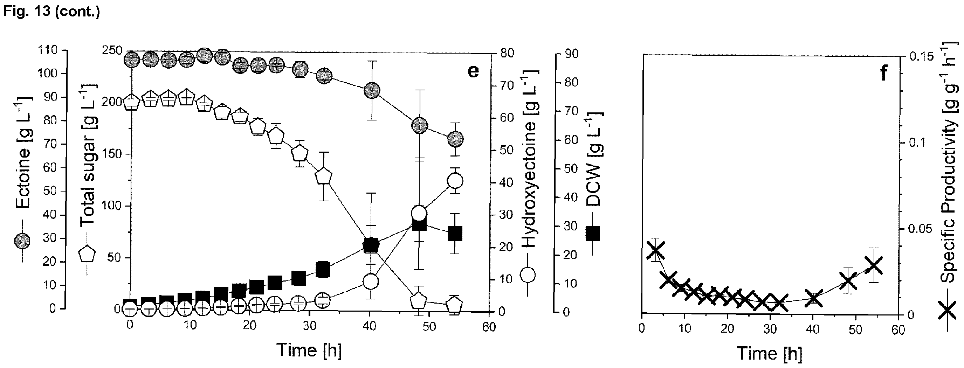

**Figure 13****:** Production of 5-hydroxyectoine in a batch process in stirred tank bioreactors using *C*. *glutamicum P_{tuf} ectD^{PST}* which episomally expressed codon optimized *ectD* from *Pseudomonas stutzeri* A1501. The strain was cultivated in batch-mode with initial sucrose and ectoine concentrations of 100 g L⁻¹ and 50 g L⁻¹ (a, b), 150 g L⁻¹ and 75 g L⁻¹ (c, d) and 200 g L⁻¹ and 100 g L⁻¹ (e, f) at 37°C. n=2.

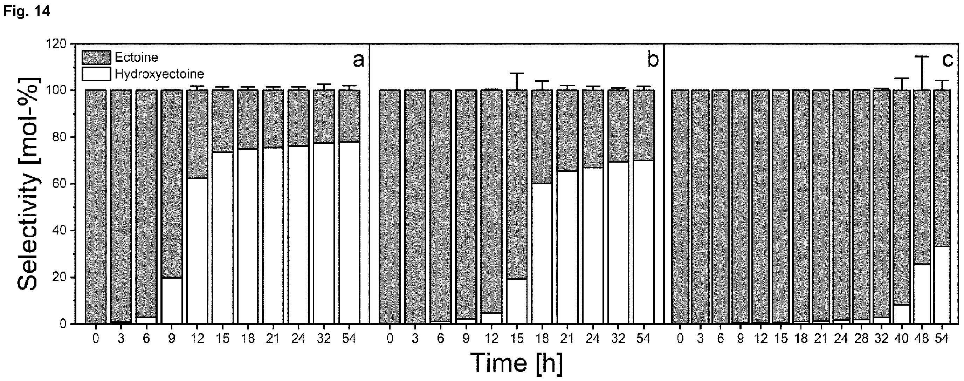

**Figure 14****:** Selectivity of 5-hydroxyectoine production in a batch process using *C*. *glutamicum P_{tuf} ectD^{PST}.* The *C*. *glutamicum* type strain ATCC 13032 episomally expressed codon optimized *ectD* from *Pseudomonas stutzeri* A1501. It was cultivated with initial sucrose and ectoine concentrations of 100 g L⁻¹ and 50 g L⁻¹ (a), 150 g L⁻¹ and 75 g L⁻¹ (b), and 200 g L⁻¹ and 100 g L⁻¹ (c) at 37°C. n=2.

### DETAILED DESCRIPTION OF THE INVENTION

A first aspect of the present invention is directed to a method for producing hydroxyectoine, comprising (a) providing a biocatalyst, wherein said biocatalyst is a recombinant bacterial host cell transformed with a promoter operably linked to a nucleotide sequence encoding an ectoine hydroxylase - ectD - (EC 1.14.11) followed by a transcriptional terminator, (b) providing a carbon source and ectoine, and (c) cultivating the bacterial host cell under suitable conditions to allow biocatalytic conversion of ectoine into hydroxyectoine, and thereby secreting the produced hydroxyectoine.

In accordance with the present invention, any suitable carbon source is envisioned in step (b) such as carbohydrates, for example sucrose, amino acids or lipids. A skilled person is able to choose a suitable carbon source for the method of the invention.

In a preferred embodiment of the present invention, it is foreseen that the nucleotide sequence encoding the ectoine hydroxylase is selected from the group consisting of *Pseudomonas stutzeri, Mycobacterium smegmatis, Halomonas elongata, Virgibacillus salexigens, Streptomyces coelicolor, Gracilibacillus sp., Acidiphilium cryptum, Alkalihalobacillus clausii, Hydrocarboniclastica marina, Methylomicrobium alcaliphilum, Neptunomonas concharum, Leptospirillum ferriphilum, Candidatus Nitrosopumilus sp., Sphyngopyxis alaskensis, Paenibacillus lautus,* and *Chromohalobacter salexigens.* According to the present invention the term "ectoine hydroxylase" is also used interchangeably with the term "ectD".

Preferably, the bacterial host cell is generally recognized as safe (GRAS) bacterial host cell. This provides the advantage that the hydroxyectoine produced according to the method of the present invention cannot be considered as harmful and thus it is possible to use the hydroxyectoine example in cosmetics.

In a preferred embodiment of the invention, it is foreseen that said bacterial host cell is selected from the group consisting of *Actinobacteria,* preferably *Actinobacteridae,* more preferably *Actinomycetales,* even more preferred *Corynebacterineae,* in particular preferred *Corynebacterium* ssp., most preferred *Corynebacterium glutamicum.*

Preferably, it is foreseen that the method is suitable to be conducted in batch mode or fed batch mode. This allows the production of hydroxyectoine in a larger scale and in a very efficient and less cost-intensive way. Presently, the prior art does not disclose any useful methods for hydroxyectoin production to be conducted in a larger scope for efficient, easy and less time, work, and cost intensive production.

In a preferred embodiment of the present invention, is foreseen that said bacterial host cell is capable of producing hydroxyectoine at a concentration in the range of about 20 g L⁻¹ to about 100 g L⁻¹, preferably of about 30 g L⁻¹ to 90 g L⁻¹, more preferably of about 40 g L⁻¹ to about 80 g L⁻¹, most preferably of about 75 g L⁻¹.

Preferably, it is foreseen according to the present invention that said bacterial host cell is capable of producing hydroxyectoine within a period of time selected from about 24 h, about 20 h, about 18 h, about 15 h, about 10 h, about 12 h, and about 8 h. Thus, the method of the present invention provides the advantage to produce hydroxyectoine within a very short time interval. Accordingly, the production of hydroxyectoine may be completed within a period of time of 24 h to 8 h or any interval between 24 h and 8 h.

In a preferred embodiment of the invention, it is foreseen that said bacterial host cell is capable of producing hydroxyectoine with a productivity of about 1.5 g/L/h to about 8 g/L/h, preferably of about 3 g/L/h to about 7.5 g/L/h, more preferably of about 4 g/L/h to about 7 g/L/h, most preferably of about 6.25 g/L/h.

Thus, the inventors were able to provide a method which allows a highly efficient hydroxyectoine production. This is demonstrated in view of the high concentration of hydroxyectoine in a short period of time. Therefore, the inventors provide a method for producing hydroxyectoine with a high productivity within a short interval. This is a crucial advantage compared to the prior art methods, such as for example described in the publication of Ma et al., 2022. Therein it has been described that a very low amount of hydroxyectoine can be produced in a simple shake flask assay in E. *coli* within a long period of time of 36 h. In view of said prior art method providing hydroxyectoine at low concentrations in a very small setting, the present method of the invention provides a novel and advantageous method which allows for the first time to produce hydroxyectoine at a large scale and in short time.

The inventors have shown that the biotransformation with an ectD from different host cells allows the provision of such a highly efficient hydroxyectoine production. This is demonstrated in the following examples, in particular in **Example 2,** and **Figures 2****,** **8** **and** **9****.** In **Example 5,** **Figure 3** the ectD performance of three strains has been investigated in more detail, namely *C. glutamicum P_{tuf}ectD^{MSM}, P_{tuf}ectD^{PST}, P_{tuf}ectD^{VSA}.* Further, in **Example 8,** **Figure 5****,** **12** the best performing strain in the shake flask, *C*. *glutamicum P_{tuf}ectD^{MSM},* has been evaluated in a fed-batch mode. This standard fed-batch configuration provided the highest hyroxyectoine titer reported so far. In a further step, the inventors investigated also the performance of *C*. *glutamicum P_{tuf}ectD^{PST}.* Advantageously, the inventors found out that said strain performed even better with a maximum hydroxyectoine titer of 42 g L⁻¹ after 22 h, surpassed that of the MSM strain by 27%. This is very surprising since in the shake flask setting the MSM strain has been identified as the best performer, as shown in **Example 3**, **Figures 2** **and** **9****.** Further, in the prior art - dissertation of Brauner, 2021, it has been reported that the ectD of P *stutzeri* may be less preferred due to expected minor stability of the enzyme. Therefore, the highly efficient production of hydroxyectoine using the ectD of *P. stutzeri* is unexpected and could not have been foreseen.

It could be shown that the method of the present invention provides also hydroxyectoine at high selectivity which means that the fraction of the target product hydroxyectoine in the final ectoine-hydroxyectoine mixture is significantly higher than the fraction of the substrate ectoine. Accordingly, the invention provides the possibility to produce ectoine and hydroxyectoine in combination. Thus, the present invention allows also the production of ectoine and hydroxyectoine in distinct mixtures. Preferably, a mixture ratio of the present invention may be a 20:80 mixture of ectoine and hydroxyectoine. Further, preferred mixtures may be 10:90, 30:70, 40:60, or 50:50 mixture of ectoine and hydroxyectoine

In a further preferred embodiment of the present invention, it is foreseen that the promoter is a constitutive promoter, and wherein the promoter is a homologous or heterologous promoter, and preferably selected from the group consisting of P_{tuf}, P_{sod} (Superoxiddismutase), P_{cspB} (surface-layer protein PS2), and P_{gapA} (glyceraldehyde 3-phosphate dehydrogenase.

Preferably, it is foreseen that the promoter operably linked to a nucleotide sequence encoding an ectoine hydroxylase - ectD - (EC 1.14.11) is comprised in a plasmid, preferably an episomal plasmid, preferably a plasmid that can replicate in *C*. *glutamicum,* wherein the plasmid is preferably selected from pClik and pCES.

In a preferred embodiment of the present invention, it is foreseen that the cultivation of the bacterial host cell in step (c) is conducted at temperatures of about 25°C to about 45°C, preferably between about 30°C to about 37°C.

Preferably, the method of the present invention foresees that the cultivation of the bacterial host cell in step (c) is conducted comprising the provision of the ectoine at a concentration of about 40 g L⁻¹ to about 120 g L⁻¹ and providing the carbon source at a concentration of about 90 g L⁻¹ to about 220 g L⁻¹.

In a further preferred embodiment of the present invention it is foreseen that instead of or additionally to step (b) the method comprises (b1) providing a further biocatalyst, wherein said further biocatalyst is a further bacterial host cell transformed with a heterologous promoter operably linked to a nucleotide sequence encoding (i) a heterologous promoter operably linked to a nucleotide sequence encoding 2,4-diaminobutyrate acetyltransferase (EC 2.3.1.178) followed by a transcriptional terminator, (ii) a heterologous promoter operably linked to a nucleotide sequence encoding L-2,4-diaminobutyrate transaminase (EC 2.6.1.76) followed by a transcriptional terminator, and (iii) a heterologous promoter operably linked to a nucleotide sequence encoding ectoine synthase (EC 4.2.1.108) followed by a transcriptional terminator; (b2) providing a carbon source, (b3) cultivating said further bacterial host cell under suitable conditions to allow biocatalytic conversion of glucose into ectoine, and preferably, (b4) separating of the further bacterial host cell.

The optional step (b1) allows the *de novo* synthesis of ectoine which may be desired in view of relative high costs of externally supplied ectoine.

Preferably, said further bacterial host cell is generally recognized as safe (GRAS) bacterial host cell.

In a preferred embodiment of the method of the invention, said further bacterial host cell is selected from the group consisting of *Corynebacterium* ssp., preferably *Corynebacterium glutamicum, Escherichia coli, Halomonas elongata, Chromohalobacter salexigens,* and *Bacillus subtilis.*

In a preferred embodiment of the present invention, it is foreseen that said nucleotide sequence encoding an ectoine hydroxylase - ectD - (EC 1.14.11) is selected from the group consisting of SEQ ID NO:7 to 38.

The nucleotide sequences encoding the ectoine hydroxylase - ectD - according to the present invention may be sequences which are native sequences of ectoine hydroxylases -ectD, or may be sequences which are codon-optimized sequences. The codon-optimized sequences may be preferably sequences which correspond to sequences strongly expressed in *Corynebacterium glutamicum.* Distinct preferred examples of native sequences of ectoine hydroxylase - ectD - are SEQ ID NO:7, 9, 11, 13, 15, 17 ,19 ,21, 23, 25, 27, 29, 31, 33, 35, and 37. Distinct preferred examples of codon-optimized sequences of ectoine hydroxylase - ectD - are SEQ ID NO:8, 10 ,12, 14 ,16, 18, 20, 22, 24, 26, 28, 30, 32, 34, 36 and 38.

Further preferred, it is foreseen that said nucleotide sequence encoding 2,4-diaminobutyrate acetyltransferase (EC 2.3.1.178) -ectA- is selected from SEQ ID NO:4.

Preferably, it is foreseen that said nucleotide sequence encoding L-2,4-diaminobutyrate transaminase (EC 2.6.1.76) - ectB - is selected from SEQ ID NO:5.

Further preferred, it is foreseen that said nucleotide sequence encoding ectoine synthase (EC 4.2.1.108) - ectC - is selected from SEQ ID NO:6.

In a further preferred embodiment of the present invention, it is foreseen that the method further comprises after step (c) the step (d) of breaking up of said bacterial host cell to recover also intracellularly produced hydroxyectoine. This optional step (d) allows a mostly complete recovery of the produced hydroxyectoine.

A second aspect of the invention is directed to a recombinant bacterial host cell comprising a promoter operably linked to a nucleotide sequence encoding an ectoine hydroxylase - ectD - (EC 1.14.11) followed by a transcriptional terminator.

In a preferred embodiment of the invention, it is foreseen that said host cell comprises a promoter as shown in SEQ ID NO:3 operably linked to a nucleotide sequence encoding an ectoine hydroxylase - ectD - (EC 1.14.11) selected from the group consisting of SEQ ID NO:7 to 38 followed by a transcriptional terminator.

Preferably, the promoter according to the present invention is a heterologous or homologous promoter as described above. According to the present invention the promoter is constitutively expressed.

In a preferred embodiment of the present invention, it is foreseen that said bacterial host cell is capable of producing hydroxyectoine at a concentration in the range of about 20 g L⁻¹ to about 100 g L⁻¹, preferably in the range of about 30 g L⁻¹ to 90 g L⁻¹, more preferably of about 40 g L⁻¹ to about 80 g L⁻¹, most preferably of about 75 g L⁻¹.

Further preferred, it is foreseen that said bacterial host cell is capable of producing hydroxyectoine in a period of time of about 10 h to 24 h, preferably of about 10 h to 20 h, more preferably of about 10 h to 15 h, most preferably of about 12 h.

Preferably, it is foreseen that said bacterial host cell is capable of producing hydroxyectoine with a productivity of about 1.5 g/L/h to about 8 g/L/h, preferably of about 3 g/L/h to about 7.5 g/L/h, more preferably of about 4 g/L/h to about 7 g/L/h, most preferably of about 6.25 g/L/h.

According to the method of the present invention it has been also possible to provide hydroxyectoine in particular with a productivity of 3,19 g/L/h within 18 h with a concentration of hydroxyectoine of 57.88 g/L in a batch process with 1.5x batch.

As already mentioned above, the inventors have been able to provide a bacterial host cell which allows the highly efficient production of hydroxyectoine. Said bacterial host cell with the advantageous productivity is unexpected and not described in the prior art.

A third aspect of the present invention is directed to the use of the recombinant bacterial host cell as described above and elsewhere in the application for the production of hydroxyectoine.

A fourth aspect of the present invention is directed to a kit comprising a bacterial host cell as described above and elsewhere in the application and a further bacterial host cell as defined above and elsewhere in the application for use in a method of the invention as described above and elsewhere in the application.

Thus, the present invention provides a kit which allows the combination of the ectoine *de novo* production with the hydroxyectoine *de novo* production.

It could be shown that the method of the present invention provides also hydroxyectoine at high selectivity which means that the fraction of the target product hydroxyectoine in the final ectoine-hydroxyectoine mixture is significantly higher than the fraction of the substrate ectoine. Accordingly, the invention provides the possibility to produce ectoine and hydroxyectoine in combination. Thus, the present invention allows also the production of ectoine and hydroxyectoine in distinct mixtures. Preferably, a mixture ratio of the present invention may be a 20:80 mixture of ectoine and hydroxyectoine. Further, preferred mixtures may be 10:90, 30:70, 40:60, or 50:50 mixture of ectoine and hydroxyectoine.

The present invention is characterized by the following items:
1. A method for producing hydroxyectoine, comprising
   (a) providing a biocatalyst, wherein said biocatalyst is a recombinant bacterial host cell transformed with a promoter operably linked to a nucleotide sequence encoding an ectoine hydroxylase - ectD - (EC 1.14.11) followed by a transcriptional terminator,
   (b) providing a carbon source and ectoine, and
   (c) cultivating the bacterial host cell under suitable conditions to allow biocatalytic conversion of ectoine into hydroxyectoine, and thereby secreting the produced hydroxyectoine.
2. The method of item 1, wherein the nucleotide sequence encoding the ectoine hydroxylase is selected from the group consisting of *Pseudomonas stutzeri, Mycobacterium smegmatis, Halomonas elongata, Virgibacillus salexigens, Streptomyces coelicolor, Gracilibacillus sp., Acidiphilium cryptum, Alkalihalobacillus clausii, Hydrocarboniclastica marina, Methylomicrobium alcaliphilum, Neptunomonas concharum, Leptospirillum ferriphilum, Candidatus Nitrosopumilus sp., Sphyngopyxis alaskensis, Paenibacillus lautus,* and *Chromohalobacter salexigens.*
3. The method of item 1 or 2, wherein said bacterial host cell is generally recognized as safe (GRAS) bacterial host cell.
4. The method of any one of the preceding items, wherein said bacterial host cell is selected from the group consisting of *Actinobacteria,* preferably *Actinobacteridae,* more preferably *Actinomycetales,* even more preferred *Corynebacterineae,* in particular preferred *Corynebacterium* ssp., most preferred *Corynebacterium glutamicum.*
5. The method of any one of the preceding items, wherein the method is suitable to be conducted in batch mode or fed batch mode.
6. The method of any one of the preceding items, wherein said bacterial host cell is capable of producing hydroxyectoine at a concentration in the range of about 20 g L⁻¹ to about 100 g L⁻¹, preferably of about 30 g L⁻¹ to 90 g L⁻¹, more preferably of about 40 g L⁻¹ to about 80 g L⁻¹, most preferably of about 75 g L⁻¹.
7. The method of any one of the preceding items, wherein said bacterial host cell is capable of producing hydroxyectoine within a period of time selected from about 24 h, about 20 h, about 18 h, about 15 h, about 10 h, about 12 h, and about 8 h.
8. The method of any one of the preceding items, wherein said bacterial host cell is capable of producing hydroxyectoine with a productivity of about 1.5 g/L/h to about 8 g/L/h, preferably of about 3 g/L/h to about 7.5 g/L/h, more preferably of about 4 g/L/h to about 7 g/L/h, most preferably of about 6.25 g/L/h.
9. The method of any one of the preceding items, wherein promoter is a constitutive promoter, and wherein the promoter is a homologous or heterologous promoter, and preferably selected from the group consisting of P_{tuf}, P_{sod} (Superoxiddismutase), P_{cspB} (surface-layer protein PS2), and P_{gapA} (glyceraldehyde 3-phosphate dehydrogenase.
10. The method of any one of the preceding items, wherein the heterologous promoter operably linked to a nucleotide sequence encoding an ectoine hydroxylase - ectD - (EC 1.14.11) is comprised in a plasmid, preferably an episomal plasmid, preferably a plasmid that can replicate in C. *glutamicum,* wherein the plasmid is preferably selected from pClik and pCES.
11. The method of any one of the preceding items, wherein the cultivation of the bacterial host cell in step (c) is conducted at temperatures of about 25°C to about 45°C, preferably between about 30°C to about 37°C.
12. The method of any one of the preceding items, wherein the cultivation of the bacterial host cell in step (c) is conducted comprising the provision of the ectoine at a concentration of about 40 g L⁻¹ to about 120 g L⁻¹ and providing sucrose at concentration of about 90 g L⁻¹ to about 220 g L⁻¹.
13. The method of any one of the preceding items, wherein instead of or additionally to step (b) the method comprises
   (b1) providing a further biocatalyst, wherein said further biocatalyst is a further bacterial host cell transformed with a heterologous promoter operably linked to a nucleotide sequence encoding
      (i) a heterologous promoter operably linked to a nucleotide sequence encoding 2,4-diaminobutyrate acetyltransferase (EC 2.3.1.178) followed by a transcriptional terminator,
      (ii) a heterologous promoter operably linked to a nucleotide sequence encoding L-2,4-diaminobutyrate transaminase (EC 2.6.1.76) followed by a transcriptional terminator, and
      (iii) a heterologous promoter operably linked to a nucleotide sequence encoding ectoine synthase (EC 4.2.1.108) followed by a transcriptional terminator;
   (b2) providing glucose,
   (b3) cultivating said further bacterial host cell under suitable conditions to allow biocatalytic conversion of glucose into ectoine, and preferably,
   (b4) separating of the further bacterial host cell.
14. The method of any one of the preceding items, wherein said further bacterial host cell is generally recognized as safe (GRAS) bacterial host cell.
15. The method of any one of the preceding items, wherein said further bacterial host cell is selected from the group consisting of *Corynebacterium* ssp., preferably *Corynebacterium glutamicum, Escherichia coli, Halomonas elongata, Chromohalobacter salexigens,* and *Bacillus subtilis.*
16. The method of any one of the preceding items, wherein said nucleotide sequence encoding an ectoine hydroxylase - ectD - (EC 1.14.11) is selected from the group consisting of SEQ ID NO:7 to 38.
17. The method of any one of the preceding items, wherein said nucleotide sequence encoding 2,4-diaminobutyrate acetyltransferase (EC 2.3.1.178) is selected from SEQ ID NO:4.
18. The method of any one of the preceding items, wherein said nucleotide sequence encoding L-2,4-diaminobutyrate transaminase (EC 2.6.1.76) is selected from SEQ ID NO:5.
19. The method of any one of the preceding items, wherein said nucleotide sequence encoding ectoine synthase (EC 4.2.1.108) is selected from SEQ ID NO:6.
20. The method of any one of the preceding items, wherein the method further comprises after step (c)
   (d) breaking up of said bacterial host cell to recover also intracellularly produced hydroxyectoine.
21. A recombinant bacterial host cell comprising a promoter operably linked to a nucleotide sequence encoding an ectoine hydroxylase - ectD - (EC 1.14.11) followed by a transcriptional terminator.
22. The recombinant bacterial host cell of item 21, wherein said host cell comprises a promoter as shown in SEQ ID NO:3 operably linked to a nucleotide sequence encoding an ectoine hydroxylase - ectD - (EC 1.14.11) selected from the group consisting of SEQ ID NO:7 to 38 followed by a transcriptional terminator.
23. The recombinant bacterial host cell of item 21 or 22, wherein said bacterial host cell is capable of producing hydroxyectoine at a concentration in the range of about 20 g L⁻¹ to about 100 g L⁻¹, preferably in the range of about 30 g L⁻¹ to 90 g L⁻¹, more preferably of about 40 g L⁻¹ to about 80 g L⁻¹, most preferably of about 75 g L⁻¹.
24. The recombinant bacterial host cell of any one of items 21 to 23, wherein said bacterial host cell is capable of producing hydroxyectoine within a period of time selected from about 24 h, about 20 h, about 18 h, about 15 h, about 10 h, about 12 h, and about 8 h.
25. The recombinant bacterial host cell of any one of items 21 to 24, wherein said bacterial host cell is capable of producing hydroxyectoine with a productivity of about 1.5 g/L/h to about 8 g/L/h, preferably of about 3 g/L/h to about 7.5 g/L/h, more preferably of about 4 g/L/h to about 7 g/L/h, most preferably of about 6.25 g/L/h.
24. Use of the recombinant bacterial host cell of any one of the items 21 to 25 for the production of hydroxyectoine.
25. Kit comprising a bacterial host cell of any one of items 21 to 25 and a further bacterial host cell as defined in any one of the items 13 to 15, and 17 to 19 for use in a method of any one of the items 1 to 20.

### EXAMPLES OF THE INVENTION

The inventors have used and conducted the following materials and methods.

### MATERIAL AND METHODS

**Microorganisms and plasmids.** *C. glutamicum* ATCC 13032 (DSM 20300) was obtained from the German Collection of Microorganisms and Cell Cultures (DSMZ, Braunschweig, Germany). For episomal expression, target genes were cloned into the shuttle vectors pClik 5α (Buschke N, Schröder H, Wittmann C: Metabolic engineering of Corynebacterium glutamicum for production of 1,5-diaminopentane from hemicellulose. Biotechnology journal 2011, 6:306-317.) and pCES-PLPV (Yim SS, Choi JW, Lee RJ, Lee YJ, Lee SH, Kim SY, Jeong KJ: Development of a new platform for secretory production of recombinant proteins in Corynebacterium glutamicum. Biotechnology and Bioengineering 2016, 113:163-172.). All strains and plasmids are listed in following **Table** .

| **Table 1. *Corynebacterium glutamicum* strains and plasmids.** | | |
|---|---|---|
| Strains/Plasmids | Description | Reference |
| Strains | | |
| E. coli DH10B | Heat shock-competent cells for vector amplification | Invitrogen |
| ATCC 13032 | Wild type of *C*. *glutamicum* | ATCC |
| *C*. *glutamicum ectABC^{opt}* | Ectoine producing strain | [15] |
| pCES | ATCC 13032 + pCES PLPV | This work |
| pCES PST | ATCC 13032 + pCES *P_{tuf} ectD_{PST}* | This work |
| pCES MSM | ATCC 13032 + pCES *P_{tuf} ectD_{MSM}* | This work |
| pCES SCO | ATCC 13032 + pCES *P_{tuf} ectD_{SCO}* | This work |
| pCES HEL | ATCC 13032 + pCES *P_{tuf} ectD_{HEL}* | This work |
| pCES VSA | ATCC 13032 + pCES *P_{tuf} ectD_{VSA}* | This work |
| pCES GSP | ATCC 13032 + pCES *P_{tuf} ectD_{GSP}* | This work |
| pCES ACR | ATCC 13032 + pCES *P_{tuf} ectD_{ACR}* | This work |
| pCES ACL | ATCC 13032 + pCES *P_{tuf} ectD_{ACL}* | This work |
| pCES HMA | ATCC 13032 + pCES *P_{tuf} ectD_{HMA}* | This work |
| pCES MAL | ATCC 13032 + pCES *P_{tuf} ectD_{MAL}* | This work |
| pCES NCO | ATCC 13032 + pCES *P_{tuf} ectD_{NCO}* | This work |
| pCES LFE | ATCC 13032 + pCES *P_{tuf} ectD_{LEE}* | This work |

| | | |
|---|---|---|
| pCES CNS | ATCC 13032 + pCES *P_{tuf} ectD_{CNS}* | This work |
| pCES SAL | ATCC 13032 + pCES *P_{tuf} ectD_{SAL}* | This work |
| pCES PLA | ATCC 13032 + pCES *P_{tuf} ectD_{PLA}* | This work |
| pCES CSA | ATCC 13032 + pCES *P_{tuf} ectD_{CSA}* | This work |
| pClik PST | ATCC 13032 + pClik *P_{tuf} ectD_{PST}* | This work |
| pClik MSM | ATCC 13032 + pClik *P_{tuf} ectD_{MSM}* | This work |
| pClik SCO | ATCC 13032 + pClik *P_{tuf} ectD_{sco}* | This work |
| pClik HEL | ATCC 13032 + pClik *P_{tuf} ectD_{HEL}* | This work |
| pClik VSA | ATCC 13032 + pClik *P_{tuf} ectD_{VSA}* | This work |

| Plasmids | | |
|---|---|---|
| pCES PLPV | Episomal vector | [77] |
| pCES P_{tuf} ectD_{PST} | Episomal vector with codon optimized *ectO* from *Pseudomonas stutzeri* A1501 under control of *Ptuf* | This work |
| pCES P_{tuf} ectD_{MSM} | Episomal vector with codon optimized *ectD* from *Mycobacterium smegmatis* ATCC 19420 under control of *Ptuf* | This work |
| pCES P_{tuf} ectD_{SCO} | Episomal vector with codon optimized *ectO* from *Streptomyces coelicolor* A3(2) under control of *Ptuf* | This work |
| pCES P_{tuf} ectD_{HEL} | Episomal vector with codon optimized *ectO* from *Halomonas elongata* ATCC 33173 under control of *Ptuf* | This work |
| pCES P_{tuf} ectD_{VSA} | Episomal vector with codon optimized *ectO* from *Virgibacillus salexigens* ATCC 700290 including amino acid exchanges A163C and S244C, under control of *Ptuf* | This work |
| pCES P_{tuf} ectD_{GSP} | Episomal vector with codon optimized *ectO* from *Gracilibacillus sp.* SCU50 under control of *Ptuf* | This work |
| pCES P_{tuf} ectD_{ACR} | Episomal vector with codon optimized *ectD* from *Acidiphilium cryptum* JF5 under control of *Ptuf* | This work |
| pCES P_{tuf} ectD_{ACL} | Episomal vector with codon optimized *ectO* from *Alkalihalobacillus clausii* 7520-2 under control of *Ptuf* | This work |
| pCES P_{tuf} ectD_{HMA} | Episomal vector with codon optimized *ectD* from *Hydrocarboniclastica marina* KCTC 62334 under control of *Ptuf* | This work |
| pCES P_{tuf} ectD_{MAL} | Episomal vector with codon optimized *ectO* from *Methylomicrobium alcaliphilum* DSM 19304 under control of *Ptuf* | This work |
| pCES P_{tuf} ectD_{NCO} | Episomal vector with codon optimized *ectO* from *Neptunomonas concharum* LHW37 under control of *Ptuf* | This work |
| pCES P_{tuf} ectD_{LFE} | Episomal vector with codon optimized *ectO* from *Leptospirillum feriiphilum* ML-04 under control of *Ptuf* | This work |
| pCES P_{tuf} ectD_{CNS} | Episomal vector with codon optimized *ectO* from *Candidatus Nitrosopumilus sp.* AR2 under control of *Ptuf* | This work |

| | | |
|---|---|---|
| pCES P_{tuf} ectD_{SAL} | Episomal vector with codon optimized *ectO* from *Sphyngopyxis alaskensis* DSM 13593 under control of *Ptuf* | This work |
| pCES P_{tuf} ectD_{PLA} | Episomal vector with codon optimized *ectO* from *Paenibacillus lautus* E7593-69 under control of *Ptuf* | This work |
| pCES P_{tuf} ectD_{CSA} | Episomal vector with codon optimized *ectO* from *Chromohalobacter salexigens* DSM 3043 under control of *Ptuf* | This work |
| pClik 5a MCS | Episomal vector | [76] |
| pClik P_{tuf} ectD_{PST} | Episomal vector with codon optimized *ectO* from *Pseudomonas stutzeri* A1501 under control of *Ptuf* | This work |
| pClik P_{tuf} ectD_{MSM} | Episomal vector with codon optimized *ectO* from *Mycobacterium smegmatis* ATCC 19420 under control of *Ptuf* | This work |
| pClik P_{tuf} ectD_{SCO} | Episomal vector with codon optimized *ectO* from *Streptomyces coelicolor* A3(2) under control of *Ptuf* | This work |
| pClik P_{tuf} *ectD_{HEL}* | Episomal vector with codon optimized *ectO* from *Halomonas elongata* ATCC 33173 under control of *Ptuf* | This work |
| pClik P_{tuf} ectD_{VSA} | Episomal vector with codon optimized *ectO* from *Virgibacillus salexigens* ATCC 700290m including amino acid exchanges A163C and S244C, under control of *Ptuf* | This work |

**Molecular design and genetic engineering.** To screen and evaluate a panel of ectoine hydroxylases, codon-optimized genes from *Mycobacterium smegmatis* ATCC 19420, *Virgibacillus salexigens* ATCC 700290 (comprising the amino acid exchanges A163C and S244C) (Widderich N, Pittelkow M, Höppner A, Mulnaes D, Buckel W, Gohlke H, Smits SHJ, Bremer E: Molecular Dynamics Simulations and Structure-Guided Mutagenesis Provide Insight into the Architecture of the Catalytic Core of the Ectoine Hydroxylase. Journal of molecular biology 2014, 426:586-600.) [, *Streptomyces coelicolorA3(2), Halomonas elongata* ATCC 33173, *Pseudomonas stutzeri* A1501, *Gracilibacillus sp.* SCU50, *Acidiphilium cryptum* JF5, *Alkalihalobacillus clausii* 7520-2, *Hydrocarboniclastica marina* KTC 62334, *Methylomicrobium alcaliphilum* DSM 19304, *Neptunomonas concharum* LHW37, *Leptospirillum ferriphilum* ML-04, *Candidatus Nitrosopumilus sp.* AR2, *Sphyngopyxis alaskensis* DSM 13593, *Paenibacillus lautus* E7593-69 and *Chromohalobacter salexigens* DSM 3043 were synthesized on basis of the digital sequence information (General Biosystems, Morrisville, NC, USA, Genscript Biotech Corp, Piscataway Township, NJ, USA). The obtained genes were amplified with specific primers (**Table 2)** and cloned each under control of the constitutive *tuf* (NCgl0480) promoter (Becker J, Klopprogge C, Zelder O, Heinzle E, Wittmann C: Amplified Expression of Fructose 1,6-Bisphosphatase in Corynebacterium glutamicum Increases In Vivo Flux through the Pentose Phosphate Pathway and Lysine Production on Different Carbon Sources. Applied and Environmental Microbiology 2005, 71:8587-8596.) [29] via Gibson Assembly (Gibson DG, Young L, Chuang R-Y, Venter JC, Hutchison Iii CA, Smith HO: Enzymatic assembly of DNA molecules up to several hundred kilobases. Nature Methods 2009, 6:343-345.) [78] into the pClik 5α and pCES-PLPV vector, cut before with *Kpn*I/*Sal*I and *Sal*I/*Xba*I, respectively. Each gene was cloned into the pCES-PLPV vector, cut before with *Sal*I and *BamH*I, under control of the *tuf promotor.* The plasmids were then amplified in *E*. *coli* DH10B, isolated and transformed via electroporation into *C*. *glutamicum* (Becker J, Buschke N, Bücker R, Wittmann C: Systems level engineering of Corynebacterium glutamicum - Reprogramming translational efficiency for superior production. Engineering in Life Sciences 2010, 10:430-438.) [79]. Generally, the amplification of DNA fragments was done using PCR (2x Phusion Flash PCR Master Mix, Thermo Fisher Scientific, Waltham, MA, USA, peQSTAR, PEQLAB Biotechnology GmbH, Erlangen, Germany). All created strains and plasmids were validated by PCR and Sanger sequencing (Genewiz, Leipzig, Germany). The sequences of the original and codon-optimized genes are given in the sequences of the sequence protocol, in particular SEQ ID NO:7 to 38.

**Table 2: Primers used for plasmid construction.**

| **Plasmid** | **Primer** | **Sequence** |
|---|---|---|
| pCES MSM | pCES MSM Ptuf fw | GGCCCCCCCTCGAGGTCGACTGGCCGTTACCCTGCGAATG |
| | pCES MSM Ptuf rv | GTGAACTGGGTGGTGGTCATTGTATGTCCTCCTGGACTTC |
| | pCES MSM ectD fw | GAAGTCCAGGAGGACATACAATGACCACCACCCAGTTCAC |
| | pCES MSM ectD rv | TGGCCGGCTGGGCCTCTAGATTAAGTCACTGGGGCCACGC |
| pCES VSA | pCES VSA Ptuf fw | GGCCCCCCCTCGAGGTCGACTGGCCGTTACCCTGCGAATG |
| | pCES VSA Ptuf rv | GATGGGTACAGGTCTTCCATTGTATGTCCTCCTGGACTTC |
| | pCES VSA ectD fw | GAAGTCCAGGAGGACATACAATGGAAGACCTGTACCCATC |
| | pCES VSA ectD rv | TGGCCGGCTGGGCCTCTAGATTAGTTCACTGCGGAGTACA |
| pCES SCO | pCES SCO Ptuf fw | GGCCCCCCCTCGAGGTCGACTGGCCGTTACCCTGCGAATG |
| | pCES SCO Ptuf rv | TCCTGGCGTGGGGTTGCCACTGTATGTCCTCCTGGACTTC |
| | pCES SCO ectD fw | GAAGTCCAGGAGGACATACAGTGGCAACCCCACGCCAGGA |
| | pCES SCO ectD rv | TGGCCGGCTGGGCCTCTAGATTACTTCACTGGGGTGAAGT |
| pCES PST | pCES PST Ptuf fw | GGGAACAAAAGCTGGGTACCTGGCCGTTACCCTGCGAATG |
| | pCES PST Ptuf rv | GGGTACAGATCTGCCTGCATTGTATGTCCTCCTGGACTTC |
| | pCES PST ectD fw | GAAGTCCAGGAGGACATACAATGCAGGCAGATCTGTACCC |
| | pCES PST ectD rv | GGCTGGGCCTCTAGAGTCGATTACAGGTACTGCTGTGGGC |
| pCES HEL | pCES HEL Ptuf fw | GGCCCCCCCTCGAGGTCGACTGGCCGTTACCCTGCGAATG |
| | pCES HEL Ptuf rv | GAGGAGGTCTGCACGGACATTGTATGTCCTCCTGGACTTC |
| | pCES HEL ectD fw | GAAGTCCAGGAGGACATACAATGTCCGTGCAGACCTCCTC |
| | pCES HEL ectD rv | TGGCCGGCTGGGCCTCTAGATTAGCCGTCTGGGGACCAGG |
| pClik MSM | pClik MSM Ptuf fw | CTGACGTCGGGCCCGGTACCTGGCCGTTACCCTGCGAATG |
| | pClik MSM Ptuf rv | GTGAACTGGGTGGTGGTCATTGTATGTCCTCCTGGACTTC |
| | pClik MSM ectD fw | GAAGTCCAGGAGGACATACAATGACCACCACCCAGTTCAC |
| | pClik MSM ectD rv | AGAAGAGCATCGATGTCGACTTAAGTCACTGGGGCCACGC |
| pClik VSA | pClik VSA Ptuf fw | CTGACGTCGGGCCCGGTACCTGGCCGTTACCCTGCGAATG |
| | pClik VSA Ptuf rv | GATGGGTACAGGTCTTCCATTGTATGTCCTCCTGGACTTC |
| | pClik VSA ectD fw | GAAGTCCAGGAGGACATACAATGGAAGACCTGTACCCATC |
| | pClik VSA ectD rv | AGAAGAGCATCGATGTCGACTTAGTTCACTGCGGAGTACA |
| pClik SCO | pClik SCO Ptuf fw | CTGACGTCGGGCCCGGTACCTGGCCGTTACCCTGCGAATG |
| | pClik SCO Ptuf rv | TCCTGGCGTGGGGTTGCCACTGTATGTCCTCCTGGACTTC |
| | pClik SCO ectD fw | GAAGTCCAGGAGGACATACAGTGGCAACCCCACGCCAGGA |
| | pClik SCO ectD rv | AGAAGAGCATCGATGTCGACTTACTTCACTGGGGTGAAGT |
| pClik PST | pClik PST Ptuf fw | CCTGACGTCGGGCCCGGTACTGGCCGTTACCCTGCGAATG |
| | pClik PST Ptuf rv | GGGTACAGATCTGCCTGCATTGTATGTCCTCCTGGACTTC |
| | pClik PST ectD fw | GAAGTCCAGGAGGACATACAATGCAGGCAGATCTGTACCC |
| | pClik PST ectD rv | CAGAAGAGCATCGATGTCGATTACAGGTACTGCTGTGGGC |
| pClik HEL | pClik HEL Ptuf fw | CTGACGTCGGGCCCGGTACCTGGCCGTTACCCTGCGAATG |
| | pClik HEL Ptuf rv | GAGGAGGTCTGCACGGACATTGTATGTCCTCCTGGACTTC |
| | pClik HEL ectD fw | GAAGTCCAGGAGGACATACAATGTCCGTGCAGACCTCCTC |
| | pClik HEL ectD rv | AGAAGAGCATCGATGTCGACTTAGCCGTCTGGGGACCAGG |

**Growth media.** Complex BHI medium (37 g L⁻¹ brain heart infusion, BHI, Becton Dickinson, Franklin Lakes, NJ, USA) was used for first pre-cultures. The medium was amended with 20 g L⁻¹ BD Difco agar (Becton Dickinson) for plate cultures. A minimal glucose medium was used for second pre-cultures and main cultures (Becker J, Klopprogge C, Wittmann C: Metabolic responses to pyruvate kinase deletion in lysine producing Corynebacterium glutamicum. Microbial Cell Factories 2008, 7:8.) [80], It contained per liter: 10 g of glucose, 1 g of NaCl, 0.055 g of Ca₂Cl₂·2H₂O, 0.2 g of MgSO₄·7H₂O, 15 g of (NH₄)₂SO₄, 24.98 g of K₂HPO₄, 7.7 g of KH₂PO₄, 20 mg of FeSO₄·7H₂O, 2 mg of FeCl₃·6H₂O, 2 mg of MnSO₄·H₂O, 1 mg of (NH₄)₆Mo₇O₂₄·4H₂O, 0.5 mg of ZnSO₄·H₂O, 0.2 mg of CuCl₂·2H₂O, 0.2 mg of Na₂B₄O₇·10H₂O, 0.5 mg of biotin, 1 mg of thiamine·HCl, 1 mg of Ca-pantothenate, and 30 mg of 3,4-dihydroxybenzoic acid. For culturing strains with episomal plasmids, 50 mg L⁻¹ of kanamycin was added from a filter-sterilized stock. Depending on the type of experiment, 99% ectoine (bitop, Dortmund, Germany) was added, as specified below. All ingredients were mixed freshly before use.

**Cultivation of *C*. *glutamicum* in shake flask.** Cultures of *C*. *glutamicum* were incubated in baffled shake flasks (10% filling volume) in an orbital shaker at 230 rpm (Multitron, Infors AG, Bottmingen, Switzerland). For pre-culturing, cells were first grown overnight in complex BHI medium using a single colony from a 2-day pre-incubated BHI agar plate as inoculum. Subsequently, cells were harvested (8500 x*g*, 5 min, RT), washed once with minimal medium, and used to inoculate a second pre-culture in minimal glucose medium. The second pre-culture was then harvested during the exponential phase (8500 x*g*, 5 min, RT), washed once, and used to inoculate the main culture, again in minimal medium. The temperature was set to 30°C for precultures and 37°C for main cultures. All experiments were conducted as triplicate.

**Production of hydroxyectoine in a two-step bioconversion process in shake flask.** In step one, *C*. *glutamicum ectABC^{opt}* (Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.) [15] was cultivated in shake flasks as described above at 30°C. After glucose depletion, cells were removed by centrifugation (10,000 x*g*, 10 min, RT). The pH of the obtained supernatant was adjusted to 7.4 (10 M NaOH) and the solution was once filtered (Filtropur S0.2, 0.2 µm, Sarstedt, Nümbrecht, Germany). Glucose was replenished to 20 g L⁻¹ from a sterile 40% stock solution. In step two, *C*. *glutamicum P_{tuf}ectD^{MSM}* was cultivated in the ectoine-containing medium from phase one in shake flasks as described above. To extract intracellular ectoine and hydroxyectoine, 1 mL culture sample was disrupted in Lysing Matrix B 2 mL tubes (MP Biomedicals, Solon, OH, USA) with a homogenisator (3x 30 s, 6500 rpm with 30 s break between cycles, Precellys^{®} 24, Bertin Technologies, Montigny-le-Bretonneux, France) at. The supernatant was collected for HPLC analysis, conducted as described above. The process was conducted in triplicate.

**Cultivation of *C*. *glutamicum* in microbioreactors.** Screening of *C. glutamicum* strains was carried out in 48-well flower plates with transparent bottom for online biomass sensing using a microbioreactor (BioLector, Beckman Coulter GmbH, Baesweiler, Germany, Baesweiler, Germany) at 1,300 rpm, 85% humidity and different temperatures, as given below. Two subsequent pre-cultures were conducted in shake flasks as described above. For main cultures, each well of the microtiter plate was filled with 1 mL of minimal glucose medium and inoculated to an initial OD₆₆₀ of 0.5. Over the course of the cultivation, biomass was measured online as light backscatter at 620 nm. All experiments were conducted as triplicate.

**Fed-batch production of hydroxyectoine in stirred tank bioreactors.** The best mutants were evaluated for their hydroxyectoine production capacity using fed-batch processes in 1 L bioreactors (SR0700DLS, DASGIP AG, Jülich, Germany). The initial batch medium (250 mL) contained per liter: 100 g of sucrose, 60 g of ectoine, 15 g of (NH₄)₂SO₄ (pH 7.0), 5 g of yeast extract (Becton Dickinson), 2.07 g of citric acid, 1.25 g of KH₂PO₄, 1.25 g of Na₂HPO₄, 1.25 g of MgSO₄·7H₂O, 170 mg of CaSO₄·2H₂O, 30 mg of ZnSO₄·7H₂O, 14 mg MnSO₄·H₂O, 100 mg of FeSO₄·7H₂O, 30 mg of pantothenic acid, 9 mg of nicotinamide, 7.5 mg of thiamine·HCl, 4.5 mg of biotin, 0.4 mg of boric acid, 0.7 mg of CuSO₄·5H₂O, 0.6 mg of CoSO₄·7H₂O, 0.5 mg of NiSO₄·6H₂O, 0.06 mg of Na₂MoO₂·2H₂O and 1 mL of antifoam 204 (Sigma-Aldrich). The process was inoculated from a pre-culture on BHI medium, supplemented with 50 µg ml⁻¹ kanamycin, and incubated at 37°C), and harvested during the mid-exponential growth phase by centrifugation (6,500 x*g*, 5 min, RT). The process was controlled at a temperature of 37°C ± 0.1. The pH was monitored online using a pH electrode (Mettler Toledo 405-DPAS-SC-K8S/225, Mettler Toledo, Giessen, Germany) and maintained at 7.0 ± 0.1 by the automatic addition of 6 M NaOH and 6 M HCl (MP8 pump system, Eppendorf, Hamburg, Germany). To provide sufficient oxygen the pO₂ level was measured online (Hamilton, Höchst, Germany) and maintained above a saturation of 30% by automatically adjusting stirrer speed and gas inflow. For data acquisition and process control the DASGIP control software was applied (DASGIP AG, Jülich, Germany).

The feed phase was initiated, when the initially added sucrose was consumed. Throughout the further process, manual feed pulses were added, when the dissolved oxygen level (pO₂) surpassed 30% which indicated substrate depletion (Rohles CM, Gläser L, Kohlstedt M, Gießelmann G, Pearson S, del Campo A, Becker J, Wittmann C: A bio-based route to the carbon-5 chemical glutaric acid and to bionylon-6,5 using metabolically engineered Corynebacterium glutamicum. Green Chemistry 2018, 20:4662-4674.) [26], The feed solution contained per liter: 600 g of sucrose, 50 g of (NH₄)₂SO₄ (pH 7.0), 5 g of yeast extract, 14 g of urea, 2.07 g of citric acid, 1.25 g of KH₂PO₄, 1.25 g of Na₂HPO₄, 1.25 g of MgSO₄·7H₂O, 170 mg of CaSO₄·2H₂O, 30 mg of ZnSO₄·7H₂O, 14 mg of MnSO₄·H₂O, 100 mg of FeSO₄·7H₂O, 30 mg of pantothenic acid, 9 mg of nicotinamide, 7.5 mg of thiamine-HCl, 4.5 mg of biotin, 0.4 mg of boric acid, 0.7 mg of CuSO₄·5H₂O, 0.6 mg of CoSO₄·7H₂O, 0.5 mg of NiSO₄·6H₂O, 0.06 of mg Na₂MoO₂·2H₂O and 1 mL of antifoam 204 (Sigma-Aldrich). The fermentations were carried out in duplicate, except for processes that served for an evaluation of alternative conditions and were conducted as single replicate each.

**Batch production of hydroxyectoine in stirred tank bioreactors.** Additionally, hydroxyectoine production was conducted in batch mode using 1 L bioreactors (SR0700DLS, DASGIP AG, Jülich, Germany). The preparation of the pre-cultures was handled as described above. Likewise, the other process settings were as described above. The batch process started with a volume of 300 mL. The standard batch medium contained per liter: 100 g sucrose, 50 g ectoine, 15 g (NH₄)₂SO₄ (pH 7.0), 5 g yeast extract, 2.07 g citric acid, 1.25 g KH₂PO₄, 1.25 g Na₂HPO₄, 1.25 g MgSO₄·7H₂O, 170 mg CaSO₄·2H₂O, 30 mg ZnSO₄·7H₂O, 14 mg MnSO₄·H₂O, 100 mg FeSO₄·7H₂O, 30 mg pantothenic acid, 9 mg nicotinamide, 7.5 mg thiamine·HCl, 4.5 mg biotin, 0.4 mg boric acid, 0.7 mg CuSO₄·5H₂O, 0.6 mg CoSO₄·7H₂O, 0.5 mg NiSO₄·6H₂O, 0.06 mg Na₂MoO₂·2H₂O and 1 mL antifoam. For testing the effect of higher sucrose and ectoine concentrations, higher concentrated batch media were used for comparison which contained 1.5-fold and 2-fold higher levels of all ingredients, respectively. All fermentations were carried out in duplicate.

**Quantification of intracellular metabolites.** To quantify intracellular amino acids and ectoines, 2 mL of mid-exponentially growing cells were sampled by fast filtration (cellulose nitrate filter, 0.2 µm, 47 mm, Sartorius, Göttingen, Germany) (Bolten CJ, Kiefer P, Letisse F, Portais J-C, Wittmann C: Sampling for Metabolome Analysis of Microorganisms. Analytical chemistry 2007, 79:3843-3849.) [81], including twice washing of the cells on the filter with two volumes of 2.5% NaCl. The filters were then transferred into cups prefilled with a 220 µM α-amino butyric acid solution as internal standard for later quantification. For metabolite extraction, the suspended filters were boiled (100°C, 15 min) and subsequently cooled on ice (2 min) before the extracts were transferred to reaction tubes and centrifuged (13,000 xg, 5 min, 4°C). The obtained supernatants were collected for analysis, and intracellular amino acids were quantified by HPLC (Hans MA, Heinzle E, Wittmann C: Quantification of intracellular amino acids in batch cultures of Saccharomyces cerevisiae. Appl Microbiol Biotechnol 2001, 56:776-779.) [82].

**Quantification of ectoine and hydroxyectoine.** Concentrations of ectoine and hydroxyectoine were analyzed by HPLC (Agilent Series 1290 Infinity, Agilent, Santa Clara, CA, USA). Separation was carried out at 20°C on a RP-phase column (Inertsil ODS-3HP, 3.0 × 150 mm, 3.0 µm, GL Sciences BV, Eindhoven, The Netherlands) using 0.1% (v/v) perchloric acid as mobile phase at a flow rate of 0.5 mL min⁻¹. The analytes were detected at a wavelength of 210 nm (Hara R, Nishikawa T, Okuhara T, Koketsu K, Kino K: Ectoine hydroxylase displays selective trans-3-hydroxylation activity towards I-proline. Applied microbiology and biotechnology 2019, 103:5689-5698.) [83]. External standards were applied for quantification.

**Quantification of sugars and organic acids.** Sugars (glucose, trehalose) and organic acids (lactate, acetate) were quantified using HPLC (Agilent Series 1260 Infinity, Agilent, Santa Clara, CA, USA) (Hoffmann SL, Kohlstedt M, Jungmann L, Hutter M, Poblete-Castro I, Becker J, Wittmann C: Cascaded valorization of brown seaweed to produce I-lysine and value-added products using Corynebacterium glutamicum streamlined by systems metabolic engineering. Metabolic Engineering 2021, 67:293-307.) [24]. The analytes were separated at 40°C by ion-moderated partitioning (Aminex HPX-87H, 300 × 7.8 mm, Bio-Rad, Hercules, CA, USA) using 5 mM H₂SO₄ as mobile phase at a flow rate of 0.5 mL min⁻¹. Detection was carried out by refractive index measurement. External standards were used for quantification.

**Quantification of amino acids.** Amino acid levels of cell lysates were quantified using HPLC (Agilent Series 1290 Infinity, Agilent, Santa Clara, CA, USA) with α-amino butyric acid as internal standard. For this, the analytes were derivatized with ortho-phthalaldehyde and 9-fluorenylmethyl chloroformate and were separated and quantified as described previously (Krömer J, Fritz M, Heinzle E, Wittmann C: In vivo quantification of intracellular amino acids and intermediates of the methionine pathway in Corynebacterium glutamicum. Analytical biochemistry 2005, 340:171-173.) [84].

### EXAMPLES

**EXAMPLE 1: *C*. *glutamicum* converts externally supplied ectoine into hydroxyectoine upon heterologous expression of the *ectD* gene from *P. stutzeri.*** As previously observed, *C*. *glutamicum* ECT-2 co-produced ectoine and hydroxyectoine upon expression of a genomic copy of the *ectABCD* operon from *P. stutzeri* (PST) (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.) [14]. This finding provided an important prerequisite for the envisioned biotransformation in the microbe: functional operation of the desired ectoine hydroxylase.

In a first experiment the inventors examined if *C*. *glutamicum,* expressing *ectD* from *P. stutzeri,* can convert externally supplied ectoine into hydroxyectoine and, beneficially, excrete the latter (**Fig. 1c**). For this purpose, the wild type was transformed with two different episomal plasmids, pClik *P_{tuf}ectD^{PST}* and pCES *P_{tuf}ectD^{PST},* differing in the plasmid backbone but each expressing the same codon-optimized *ectD* under control of *P_{tuf},* the native promoter of the *tuf* gene, enabling strong constitutive expression (Becker J, Klopprogge C, Zelder O, Heinzle E, Wittmann C: Amplified Expression of Fructose 1,6-Bisphosphatase in Corynebacterium glutamicum Increases In Vivo Flux through the Pentose Phosphate Pathway and Lysine Production on Different Carbon Sources. Applied and Environmental Microbiology 2005, 71:8587-8596. Becker J, Zelder O, Häfner S, Schröder H, Wittmann C: From zero to hero-Design-based systems metabolic engineering of Corynebacterium glutamicum for I-lysine production. Metabolic Engineering 2011, 13:159-168.) [29, 30]. The two mutants were grown in a miniaturized and parallelized reactor system. After 24 hours of incubation at 30°C in minimal glucose medium, supplemented with 1 mM ectoine, hydroxyectoine, however, was not detected, suggesting on a first glance that the bioconversion had failed (**Fig. 1d**). Surprisingly, ectoine was virtually not detected too. The solute was apparently taken up. Because the cells had no capacity to degrade ectoine, it therefore appeared possible that the desired ectoine hydroxylation took place inside the cells. However, even this had happened, the product was apparently not secreted, although the microbe was basically capable to export it (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.) [14].

When evaluated at higher initial ectoine level (5, 10, 15 mM), both strains, beneficially, secreted hydroxyectoine into the medium (**Fig. 1d**). Approximately 20 - 30% of the added ectoine was converted within 24 h. For initially 5 mM ectoine as example, up to 1.5 mM hydroxyectoine was secreted. Notably, in all cases, a substantial amount of the initially supplied ectoine could not be recovered. Both plasmids worked equally well. Cell fitness was not affected in any of the set-ups, as the specific growth rate remained constant throughout. Overall, the results provided an important proof of concept, but the conversion efficiency was rather low. The 30% hydroxyectoine content, achieved at best, left space for further optimization.

**EXAMPLE 2: Alternative ectoine hydroxylases from** ***M. smegmatis, H. elongata,** and V.* ***salexigens* enable hydroxyectoine production in *C*. *glutamicum.*** To improve the conversion, the inventors tested a diverse set of other EctD variants for their performance. As donors for EctD, the inventors selected the halophilic microbes *Halomonas elongata* (HEL), the industrial ectoine producer (Kunte HJ, Lentzen G, Galinski E: Industrial Production of the Cell Protectant Ectoine: Protection Mechanisms, Processes, and Products. Current Biotechnology 2014, 3:10-25.) [10], and *Virgibacillus salexigens* (VSA), naturally accumulating mixtures of ectoine and hydroxyectoine (Bursy J, Pierik AJ, Pica N, Bremer E: Osmotically Induced Synthesis of the Compatible Solute Hydroxyectoine Is Mediated by an Evolutionarily Conserved Ectoine Hydroxylase. Journal of Biological Chemistry 2007, 282:31147-31155.) [31]. For the latter, the inventors focused on a mutated EctD version that had shown superior kinetic properties *in vitro* (Widderich N, Pittelkow M, Höppner A, Mulnaes D, Buckel W, Gohlke H, Smits SHJ, Bremer E: Molecular Dynamics Simulations and Structure-Guided Mutagenesis Provide Insight into the Architecture of the Catalytic Core of the Ectoine Hydroxylase. Journal of molecular biology 2014, 426:586-600.) [32], Furthermore, two Gram-positive natural (hydroxy)ectoine-producers were chosen because of their taxonomically close relationship to *C. glutamicum: Mycobacterium smegmatis* (MSM) (Ofer N, Wishkautzan M, Meijler M, Wang Y, Speer A, Niederweis M, Gur E: Ectoine biosynthesis in Mycobacterium smegmatis. Applied and Environmental Microbiology 2012, 78:7483-7486.) [33] and *Streptomyces coelicolor* (SCO) (Bursy J, Kuhlmann AU, Pittelkow M, Hartmann H, Jebbar M, Pierik AJ, Bremer E: Synthesis and uptake of the compatible solutes ectoine and 5-hydroxyectoine by Streptomyces coelicolor A3(2) in response to salt and heat stresses. Applied and Environmental Microbiology 2008, 74:7286-7296.) [34] (**Fig. 8**). Codon-optimization of the *ectD* genes, cloning into the two vectors under control of *P_{tuf},* strain construction, and strain evaluation at different ectoine levels was carried out as before. Beneficially, most EctD variants enabled hydroxyectoine production (**Fig. 2a**). The pCES MSM, VSA, and HEL variants accumulated up to 1.0 mM, 0.7 mM, and 0.2 mM of the product, respectively. As exception, strains that expressed the SCO enzyme variant did not yield any hydroxyectoine. The latter eventually related to general difficulties in expressing genes from *Streptomyces* in other hosts or to an incompatibility of the enzyme with the chosen culture. Again, batches with 1 mM of ectoine had mostly no solutes left in the cultivation broth. An exception was observed for the EctD variant from *H. elongata,* which resulted in small amounts of hydroxyectoine in the medium, and EctD from *S*. *coelicolor,* where minor shares of ectoine were observed. As before, cell fitness remained unchanged over the increasing ectoine concentration. Strains expressing *ectD* on the episomal pClik plasmid performed similar to the ones that carried the pCES-based strains (**Fig. 9a**). The evaluation broadened the collection of suitable EctD proteins for further optimization.

**EXMPLE 3: Increased temperature boosts hydroxyectoine production up to 97% yield.** The initial conversions were done at 30°C, the optimum growth temperature of *C*. *glutamicum* (Leszczewicz M, Walczak P: Selection of Thermotolerant Corynebacterium glutamicum Strains for Organic Acid Biosynthesis. Food Technology and Biotechnology 2019, 57:249-259.) [35], Enzymes, however, have their optimal temperature mostly between 30 to 40°C (Widderich N, Höppner A, Pittelkow M, Heider J, Smits SHJ, Bremer E: Biochemical properties of ectoine hydroxylases from extremophiles and their wider taxonomic distribution among microorganisms. PloS one 2014, 9:e93809.) [36], Notably, hydroxyectoine is preferably formed at higher temperature, and this effect was also observed in *C*. *glutamicum* before (Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.) [14]. Therefore, additional cultivations were conducted at 33°C and 37°C. Higher temperature strongly increased hydroxyectoine production (**Fig. 2b****,c**). The MSM variant showed the overall best performance. At 37°C it produced hydroxyectoine with 97% and 82% selectivity out of 1 and 5 mM supplied ectoine, respectively. The PST variant performed similarly well in presence of 5 mM of ectoine. Both, the HEL and VSA variants showed high selectivity for 1 mM of ectoine but did not scale up accordingly at higher level. At 37°C, slight hydroxyectoine formation was also observed for the SCO variant, demonstrating that also this enzyme was functionally expressed in *C*. *glutamicum,* although its activity was far below that of the other candidates. Generally, increased temperature resulted in impaired cell viability and a declining growth rate (**Fig. 2b****,c**)**.** As before, the final titer of both ectoines did not sum up to the initially supplied concentration. Up to 1.5 mM of the overall pool was no longer detectable in the supernatant. Again, the overall performance was comparable between pClik- and pCES-based producers (**Fig. 9b****,c**). All following experiments were conducted using pCES strains.

**EXAMPLE 4: Under optimum conditions, ten out of eleven further ectoine hydroxylases from phylogenetically diverse bacterial and archaeal donors are suitable for hydroxyectoine production.** Beyond the five tested *ectD* candidates, ectoine hydroxylases had been identified in 272 out of 6,428 bacterial genomes, indicating a wide distribution within the bacterial kingdom (Widderich N, Höppner A, Pittelkow M, Heider J, Smits SHJ, Bremer E: Biochemical properties of ectoine hydroxylases from extremophiles and their wider taxonomic distribution among microorganisms. PloS one 2014, 9:e93809.) [36], The inventors were interested, if recombinant hydroxyectoine production in *C*. *glutamicum* would be restricted to selected EctD variants or if the strategy would (beneficially) work more generally. Using the optimal setup (5 mM ectoine, 37°C), the inventors evaluated a broader range of *ectD* variants from different bacterial and archaeal families: *Bacillaceae* (*Gracilibacillus sp.* SCU50, GSP; *Alkalihalobacillus clausii* 7520-2, ACL), *Paenibacillaceae* (*Paenibacillus lautus* E7593-6, PLA), Acetobacteraceae (*Acidiphilium cryptum* JF5, ACR), *Alteromonadaceae (Hydrocarboniclastica marina* KCTC 62334, HMA), Methylococcaceae (*Methylomicrobium alcaliphilum* DSM 19304, MAL), *Oceanospirillaceae* (*Neptunomonas concharum* LHW37, NCO), *Nitrospiraceae* (*Leptospirillum ferriphilum* ML-04, LFE), *Sphingomonadaceae* (*Sphingopyxis alaskensis* DSM 13593, SAL), Halomonadaceae (*Chromohalobacter salexigens* DSM 3043, CSA), and the archaeal family *Nitrosopumilaceae* (*Candidatus nitrosopumilus sp.* AR2 (CNS). Surprisingly, ten out of the eleven enzyme variants led to hydroxyectoine production (**Fig. 2d**). Even the archaeal gene, being phylogenetically highly different, enabled high-efficiency hydroxyectoine production. Among all *ectD* variants tested, the genes from the gram-positive bacteria *A*. *clausii* 7520-2, P. *lautus* E7593-6, and M. *smegmatis,* and that of the gram-negative *A*. *cryptum* JF5 and P *stutzeri* worked best. Considering also the first screening round with four out of five genes that successfully worked, the strategy appeared generally feasible, almost regardless of the origin of the gene.

**EXAMPLE 5: Efficient hydroxyectoine synthesis requires well-growing cells.** For further investigation, three strains *C. glutamicum P_{tuf}ectD^{MSM}, P_{tuf}ectD^{PST}, P_{tuf}ectD^{VSA}* were monitored in shake flasks over time, using 37°C with supplementation of 5 mM ectoine. Cells grew exponentially until glucose, the carbon and energy source, was fully consumed which happened after 15 to 17 h (**Fig. 3**). Ectoine conversion into hydroxyectoine started immediately and was growth-associated. After growth had stopped, cells continued to secrete hydroxyectoine at reduced level. However, ectoine was no longer consumed at this stage indicating that the increase was eventually due to an extra release from the cells. It was interesting to note that the three strains strongly differed in their production performance. The highest hydroxyectoine titer (2.8 mM) was achieved using the MSM-based EctD variant, 50% more than for the EctD from *P. stutzeri* and even 100% more than for the mutated EctD from *V*. *salexigens.* When inspecting the yield, i. e. produced hydroxyectoine versus consumed ectoine as estimated from supernatant analysis, (**Fig. 3d****-f**), it turned out that none of the cell factories achieved the expected 1:1 ratio. In fact, the yield ranged from 0.51 over 0.68 to 0.74 mol mol⁻¹ for the VSA, PST, and MSM strains, respectively. The correlation was linear, except for the final stage of the process, where an increase in the hydroxyectoine level was linked to a rather constant ectoine level.

**EXAMPLE 6: Ectoines are highly enriched inside the cells, whereby hydroxyectoine is preferred over ectoine.** As a substantial fraction of the initially supplied ectoine was apparently missing at the end of the process while *C*. *glutamicum* was obviously not capable of degrading it, the inventors evaluated if the solute (and also its derivative hydroxyectoine) remained inside the cells. In addition, the inventors searched for other metabolic changes, observed before e. g. in *Halomonas* sp. SBS 10 (Kushwaha B, Jadhav I, Verma HN, Geethadevi A, Parashar D, Jadhav K: Betaine accumulation suppresses the de-novo synthesis of ectoine at a low osmotic concentration in Halomonas sp SBS 10, a bacterium with broad salinity tolerance. Molecular biology reports 2019, 46:4779-4786.) [37], *E*. *coli* (Wittmann C, Weber J, Betiku E, Krömer J, Bohm D, Rinas U: Response of fluxome and metabolome to temperature-induced recombinant protein synthesis in Escherichia coli. J Biotechnol 2007, 132:375-384.) [38], and *B. subtilis* (Kohlstedt M, Sappa PK, Meyer H, Maaß S, Zaprasis A, Hoffmann T, Becker J, Steil L, Hecker M, van Dijl JM, et al: Adaptation of Bacillus subtilis carbon core metabolism to simultaneous nutrient limitation and osmotic challenge: a multi-omics perspective. Environmental microbiology 2014, 16:1898-1917.) [39] and providing valuable insights under genetic and environmental perturbation.

For this purpose, the inventors extracted and quantified intracellular ectoine, hydroxyectoine, and free proteinogenic amino acids from the three strains during mid exponential growth, i. e. after 10 h (**Fig. 3**). *C*. *glutamicum,* carrying the empty pCES plasmid, served as reference. As expected, the glucose-grown control strain without ectoine addition did not contain intracellular ectoine and hydroxyectoine (**Fig. 4**). When cultivated on glucose in presence of 5 mM ectoine, however, *C*. *glutamicum* pCES exhibited a high intracellular ectoine level of around 200 µmοl g_{CDW}⁻¹. Obviously, the strain efficiently took up the solute. The three ectoine hydroxylase-expressing strains pCES PST, MSM, and VSA, grown on glucose in the presence of ectoine, showed another, even more interesting picture. They all contained both, ectoine and hydroxyectoine. Remarkably, intracellular hydroxyectoine occurred at a huge level of 280 to 320 µmοl g_{CDW}⁻¹. In comparison, ectoine was present at a much lower level of only 20 µmοl g_{CDW}⁻¹ and thus accounted for only 5% of the total pool of the two ectoines. This finding was remarkable, because outside of the cells, ectoine was in excess at this stage of the culture (**Fig. 3**).

With regard to production, the intracellular pool displayed a substantial fraction of the totally synthetized hydroxyectoine. Considering this pool (and also the remaining intracellular ectoine) together with the extracellular levels provided the truly achieved yield of the conversion. When calculating this value, 100% conversion yield was obtained for all strains, i. e. each converted ectoine molecule yielded one hydroxyectoine molecule as expected from the pathway stoichiometry (**Fig. 3d****-f**)**.** It should be noted that, because of the strong intracellular accumulation, 25 to 50% of the product was kept inside the cells, suggesting breaking up the cells at the end of the process in order to collect all product formed.

**EXAMPLE 7: High intracellular levels of the ectoines coincide with decreased intracellular pools of amino acids of the α-ketoglutarate family.** The accumulation of ectoine and hydroxyectoine inside the cells had consequences for the intracellular abundance of amino acids from the α-ketoglutarate family (**Fig. 4**). Already when adding ectoine to the non-producing control strain that carried the empty pCES vector, the pools of I-glutamic acid, I-glutamine, and I-proline were strongly reduced. The decline was also obvious for the three hydroxyectoine producers, with I-glutamic acid being reduced by up to 47% in the MSM strain. Likewise, intracellular levels of I-glutamine (up to 61%) and I-proline (up to 50%) dropped substantially. A similar pattern was found for I-histidine, where the concentrations were halved in the presence of solutes, while other amino acids did not change significantly in their abundance between strain and condition (**Fig. 4****,** **Fig. 10**).

**EXAMPLE 8: Benchmarking** ***C. glutamicum** P_{tuf}ectD^{MSM}* **in a fed-batch process provides hydroxyectoine at high level from sucrose and ectoine.** The best performing strain in shake flask, *C*. *glutamicum P_{tuf}ectD^{MSM},* was now evaluated in fed-batch mode (**Fig. 5a**). The process started from an initial level of 100 g L⁻¹ of sucrose (as growth substrate), 60 g L⁻¹ of ectoine (as precursor for the biotransformation), and 5 g L⁻¹ yeast extract (to support cell vitality at 37°C). The cells quickly consumed sucrose. The high sugar level used, obviously did not cause any inhibitory effect on cell growth. Hydroxyectoine was produced from early on and reached a level of 29 g L⁻¹ after 15 h. Within this time, the batched sugar was almost completely consumed, and feeding was started at constant rate from a concentrated feed that contained 600 g L⁻¹ sucrose plus a minor share of yeast extract (5 g L⁻¹) which kept the sugar level in the bioreactor at about 10 g L⁻¹, while the dissolved oxygen level was controlled at 30% saturation. During feed phase, cell growth continued, and biomass reached a maximum of 60 g_{CDW} L⁻¹ after 30 h. The hydroxyectoine level increased further during the initial feed period. A maximum hydroxyectoine titer of 33 g L⁻¹ was reached after 26 h. Although the biotransformation continued further on, as visible from the still strong use of ectoine during this phase, the actual concentration of hydroxyectoine decreased during later stages, caused by the overlay of the weaker production with the dilution resulting from the added feed. However, the on-going conversion further enhanced the selectivity between ectoine and hydroxyectoine. Finally, hydroxyectoine was obtained at 79 mol-% selectivity.

In a series of further fermentations, the inventors investigated other process conditions to eventually improve production performance (**Fig. 12**). Two scenarios aimed at an increased supply of co-substrates required by the EctD protein, namely oxygen and I-glutamic acid (Czech L, Hermann L, Stöveken N, Richter AA, Höppner A, Smits SHJ, Heider J, Bremer E: Role of the Extremolytes Ectoine and Hydroxyectoine as Stress Protectants and Nutrients: Genetics, Phylogenomics, Biochemistry, and Structural Analysis. Genes 2018, 9:177.) [19]. Neither supplementation with I-glutamic acid during the batch or the feed phase nor the control of dissolved oxygen DO at a higher level of 60% was found beneficial. In fact, production remained rather unchanged. Likewise, the attempt to limit growth by omitting yeast extract and reducing nitrogen supply and, in this way, channel more cellular resources towards product formation, was not found successful. The retarded growth even negatively affected hydroxyectoine formation. A fourth set-up batched ectoine at only 10 g L⁻¹ and then added periodic feed pulses to keep its concentration above 5 g L⁻¹. It was, however, also less efficient and yielded only 19 g L⁻¹ hydroxyectoine. In this way, the inventors could successfully demonstrate conversion of ectoine into hydroxyectoine at higher scale. The standard fed-batch configuration provided the highest hydroxyectoine titer, reported to date.

**EXAMPLE 9: *C*. *glutamicum P_{tuf}ectD^{PST}* accumulates 42 g L⁻¹ hydroxyectoine in a fed-batch process.** After bench-marking the *C*. *glutamicum P_{tuf}ectD^{MSM}* strain, the inventors wanted to also investigate the performance of the second-best strain *C*. *glutamicum P_{tuf}ectD^{PST}* in fed-batch mode (**Fig. 5b**). The setup remained the same. As before, cells quickly consumed sucrose and feeding was started after 15 h to maintain the sugar level above 10 g L⁻¹. At that time, 30 g L⁻¹ of hydroxyectoine were present, comparable to the MSM strain. Likewise, biomass concentration rose up to 60 g_{CDW} L⁻¹ after 30 h during the feed phase. However, hydroxyectoine production was comparably higher during the initial feed phase, with a titer of 41 g L⁻¹ after 18 h. Due to the still on-going production with ectoine running short the inventors added an additional ectoine shot after 19 h, to eventually maximize the outcome of the process. Over the next 20 h, the bioconversion of ectoine continued, however, with a constantly declining turnover rate. At last, selectivity of hydroxyectoine was 75 mol-%. The maximum hydroxyectoine titer of 42 g L⁻¹ after 22 h, surpassed that of the MSM strain by 27%.

**EXAMPLE 10: In batch fermentation, driven by high substrate levels, *C*. *glutamicum P_{tuf}ectD^{PST}* provides hydroxyectoine at a titer of 74 g L⁻¹ and a selectivity of 70%.** Albeit fed-batch fermentation led to the high hydroxyectoine titers, the feed part of the process added only slight benefits for the bioconversion. The batch phase, lasting only for one third of the total process time, provided already up to 87% of the maximum titer. Beneficially, the feed phase increased selectivity, but prolonged the process and later even diluted the product. Hence, the inventors aimed to upgrade the process to be run faster and simpler. Inspired by the excellent tolerance of engineered *C*. *glutamicum* which efficiently used high sucrose levels, the inventors shifted to a batch mode operation that was driven by high substrate levels. Three conditions that used different starting concentrations of sucrose and ectoine were evaluated: (i) 100 g L⁻¹ and 50 g L⁻¹ (100%), (ii) 150 g L⁻¹ and 75 g L⁻¹ (150%), and (iii) 200 g L⁻¹ and 100 g L⁻¹ (200%), respectively. To match the higher concentrations of sucrose and ectoine, all other components were increased to 150% and 200%, as compared to the first condition. In all scenarios, *C*. *glutamicum P_{tuf}ectD^{PST}* was used (**Fig. 5c****,d,** **Fig. 12**).

The 100%-batch process started at 100 g L⁻¹ sucrose and 50 g L⁻¹ ectoine. Growth of strain *P_{tuf}ectD^{PST}* set in immediately. The cells grew at a high rate of 0.41 h⁻¹, which was even higher than that observed for the exponentially growing strain under moderate substrate levels (**Fig. 2b****,c**)**.** Accordingly, sucrose was consumed very fast. The sugar was depleted already after 12 h. At this time point, the hydroxyectoine titer had reached 37 g L⁻¹, meaning that 60% of the ectoine had been converted. Remarkably, production continued after the cells had stopped growing and entered the stationary phase so that the hydroxyectoine titer increased to 48 g L⁻¹ after 21 h which was 15% higher than the maximum value observed in the fed-batch before. The desired derivative was present at 78% selectivity (**Table** , **Fig. 14a**)**.**

**Table 3: Kinetics and stoichiometry of hydroxyectoine production from ectoine in C. glutamicum P_{tuf}ectD^{PST} during batch fermentation with differently concentrated media. n=3.**

| **Strain** | **1-fold** | **1.5-fold** | **2-fold** |
|---|---|---|---|
| µₘₐₓ [h⁻¹] | 0.41 ± 0.01 | 0.25 ± 0.00 | 0.08 ± 0.01 |
| Y_{X/Suc} [g g⁻¹] | 0.32 ± 0.02 | 0.30 ± 0.00 | 0.13 ± 0.00 |
| q_{EctOH} [g g⁻¹ h⁻¹] | 0.12 ± 0.00 | 0.10 ± 0.01 | 0.04 ± 0.00 |
| Y_{EctOH/Ect} [mol mol⁻¹] | 0.93 ± 0.03 | 0.99 ± 0.07 | 1.00 ± 0.04 |
| EctOHₘₐₓ [g L⁻¹] | 48.19 ± 0.52 | 74.42 ± 3.84 | 40.48 ± 4.00 |
| Selectivity_{EctOH} [%] | 78.08 ± 2.04 | 70.05 ± 1.75 | 33.15 t 1.76 |

Pleasingly, the 150%-batch process turned out to be even more efficient. Despite the higher sucrose level of 150 g L⁻¹, cells started growth without any lag phase. The growth rate was slightly reduced to 0.25 h⁻¹, so that the depletion of the sugar lasted somewhat longer than that in the lower concentrated batch, approximately 18 h. At this time point, the engineered cell factory had accumulated 58 g L⁻¹ hydroxyectoine, and the product titer continued to rise even further later on. Finally, 74 g L⁻¹ hydroxyectoine was obtained at a selectivity of 70% after 54 h **(Table** , **Fig. 14b****).** The most concentrated batch was less efficient (**Fig. 12e,f).** Cell growth was rather low (0.08 h⁻¹) so that the sucrose depletion took 54 h, far longer than for the other set-ups. Here, only 40 g L⁻¹ hydroxyectoine was secreted at a lower selectivity of 33 mol-% (**Table** , **Fig. 14c**). Obviously here, the substrate levels were so high that they reduced the vitality and metabolic activity of the cells.

**EXAMPLE 11: A two-step bioprocess with two complementary *C. glutamicum* cell factories allows** *de-novo* **production of hydroxyectoine from sugar.** Towards, a more cost effective and sustainable process, the inventors aimed for hydroxyectoine production from sugar as the sole raw material, without supplementation of pre-purified ectoine. The strategy involved a two-step fermentation setup, with *de novo* ectoine synthesis in the first step and ectoine bioconversion into hydroxyectoine in the second step, directly in the broth of the first fermentation. The inventors applied the recently created cell factory *C*. *glutamicum ectABC^{opt}* (Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.) [15] to produce ectoine in step one. Grown on minimal glucose medium (10 g L⁻¹) at 30°C, the strain accumulated 12.8 mM of ectoine within 24 h and, thereby, achieved a high yield of 0.23 mol mol⁻¹ (**Fig. 6a**). Subsequently, the cells were removed by centrifugation and filtration. The pH of the obtained supernatant was adjusted to 7.4 and the medium was replenished with 20 g L⁻¹ of glucose. Then, the medium was inoculated with *C*. *glutamicum P_{tuf}ectD^{MSM}* and incubated further at 37°C. Within 30 h, a significant fraction of hydroxyectoine was formed and secreted (**Fig. 6b****, Table 1**). To recover intracellular product, cells were finally disrupted, increasing the final titer by 74% to 5.1 mM (**Fig. 6c**).

**Table 4: Kinetics and stoichiometry of 5-hydroxyectoine of a one-step production from glucose and ectoine in C. glutamicum P_{tuf}ectD^{PST}, P_{tuf}ectD^{MSM}, P_{tuf}ectD^{VSA}, referring to the batch cultures in Fig. 3. A non-producing control strain containing the empty plasmid is shown for comparison. Furthermore, the data in the right column show the performance of the second step of a two-step process (Fig. 7), which is based on fermented medium with de novo pre-synthetized ectoine, freshly supplemented with glucose. n=3.**

| **Strain** | **One-step biotransformation** | | | | **Two-step de *novo* synthesis** |
|---|---|---|---|---|---|
| | **Control** | **PST** | **VSA** | **MSM** | **MSM** |
| µₘₐₓ [h⁻¹] | 0.38 ± 0.05 | 0.23 ± 0.02 | 0.19 ± 0.02 | 0.17 ± 0.01 | 0.19 ± 0.00 |
| Y_{X/Glc} [g mol⁻¹] | 62.20 ± 2.15 | 73.07 ± 3.06 | 69.73 ± 2.74 | 63.30 ± 1.90 | 68.23 ± 1.33 |
| Y_{EctOH/Ect} [mol mol⁻¹] | n.d. | 0.68 ± 0.01 | 0.51 ± 0.01 | 0.74 ± 0.03 | 0.56 ± 0.02 |
| EctOHₘₐₓ [mmol L⁻¹] | n.d. | 1.88 ± 0.00 | 1.38 ± 0.01 | 2.82 ± 0.11 | 2.93 ± 0.10 |
| Selectivity_{EctOH} [%] | n.d. | 40.72 ± 0.12 | 33.21 ± 0.29 | 62.36 ± 0.34 | 32.30 ± 1.06 |
| Y_{Tre/Glc} [mmol mol⁻¹] | 4.97 ± 0.12 | 1.88 ± 0.00 | 6.13 ± 0.15 | 5.97 ± 0.15 | 6.40 ± 0.20 |

The experimental results of the present invention may be summarized and assessed according to the following. ***C*. *glutamicum P_{tuf}ectD^{PST}* sets a milestone towards industrial hydroxyectoine production.** Hydroxyectoine, the hydroxylated derivative of the industrial flagship ectoine seems to become the next star on the extremolyte market, given its unique properties (Smiatek J, Harishchandra RK, Rubner O, Galla H-J, Heuer A: Properties of compatible solutes in aqueous solution. Biophysical chemistry 2012, 160:62-68. Smiatek J, Harishchandra RK, Galla H-J, Heuer A: Low concentrated hydroxyectoine solutions in presence of DPPC lipid bilayers: A computer simulation study. Biophysical chemistry 2013, 180-181:102-109**.** Smiatek J: Osmolyte Effects: Impact on the Aqueous Solution around Charged and Neutral Spheres. The Journal of Physical Chemistry B 2014, 118:771-782. Herzog M, Dwivedi M, Kumar Harishchandra R, Bilstein A, Galla H-J, Winter R: Effect of ectoine, hydroxyectoine and β-hydroxybutyrate on the temperature and pressure stability of phospholipid bilayer membranes of different complexity. Colloids and Surfaces B: Biointerfaces 2019, 178:404-411.**)** [7, 40-42] and the proven economic value for these type of products, paved through ectoine (Becker J, Wittmann C: Microbial production of extremolytes - high-value active ingredients for nutrition, health care, and well-being. Current opinion in biotechnology 2020, 65:118-128.) [1]. As shown here, the expression of a codon-optimized copy of *ectD,* encoding ectoine hydroxylase, in wild type of *C*. *glutamicum,* supported by bioprocess development, enabled high-level production of hydroxyectoine. *C*. *glutamicum* is a well-known, safe, and industrially proven host (Rohles CM, Pauli S, Giesselmann G, Kohlstedt M, Becker J, Wittmann C: Systems metabolic engineering of Corynebacterium glutamicum eliminates all by-products for selective and high-yield production of the platform chemical 5-aminovalerate. Metabolic Engineering 2022, 73:168-181. Becker J, Rohles CM, Wittmann C: Metabolically engineered Corynebacterium glutamicum for bio-based production of chemicals, fuels, materials, and healthcare products. Metabolic Engineering 2018, 50:122-141. Wolf S, Becker J, Tsuge Y, Kawaguchi H, Kondo A, Marienhagen J, Bott M, Wendisch Volker F, Wittmann C: Advances in metabolic engineering of Corynebacterium glutamicum to produce high-value active ingredients for food, feed, human health, and well-being. Essays in Biochemistry 2021, 65:197-212. Weiland F, Kohlstedt M, Wittmann C: Guiding stars to the field of dreams: Metabolically engineered pathways and microbial platforms for a sustainable lignin-based industry. Metabolic Engineering 2022, 71:13-41. Kind S, Jeong WK, Schröder H, Wittmann C: Systems-wide metabolic pathway engineering in Corynebacterium glutamicum for bio-based production of diaminopentane. Metabolic Engineering 2010, 12:341-351. Kind S, Kreye S, Wittmann C: Metabolic engineering of cellular transport for overproduction of the platform chemical 1,5-diaminopentane in Corynebacterium glutamicum. Metabolic Engineering 2011, 13:617-627. Rohles CM, Gießelmann G, Kohlstedt M, Wittmann C, Becker J: Systems metabolic engineering of Corynebacterium glutamicum for the production of the carbon-5 platform chemicals 5-aminovalerate and glutarate. Microbial Cell Factories 2016, 15:154.) [27, 43-48]. The microbe produces no endotoxins, does not undergo phage lysis, and is generally recognized as safe (GRAS), allowing the synthesis of a range of commercial products granted GRAS status by the United States Food and Drug Administration for the food and pharmaceutical industries (Wolf S, Becker J, Tsuge Y, Kawaguchi H, Kondo A, Marienhagen J, Bott M, Wendisch Volker F, Wittmann C: Advances in metabolic engineering of Corynebacterium glutamicum to produce high-value active ingredients for food, feed, human health, and well-being. Essays in Biochemistry 2021, 65:197-212.) [44], which appears as a great benefit towards the safe, industrial-scale production of hydroxyectoine as active ingredient for cosmetic and pharmaceutical application.

During development, the mixed outcome from the evaluation of ectoine hydroxylases from several donors (**Fig. 2****,** **Fig. 3****, Table 4**) underlines the importance to screen different variants at the start (Widderich N, Höppner A, Pittelkow M, Heider J, Smits SHJ, Bremer E: Biochemical properties of ectoine hydroxylases from extremophiles and their wider taxonomic distribution among microorganisms. PloS one 2014, 9:e93809.) [36], because only a few candidates matched with the cellular machinery of the host (Czech L, Stöveken N, Bremer E: EctD-mediated biotransformation of the chemical chaperone ectoine into hydroxyectoine and its mechanosensitive channel-independent excretion. Microbial Cell Factories 2016, 15:126.) [23], Hereby, the successful down-scaling of the process to microtiter plate scale at high reproducibility (**Fig. 1d****,** **Fig. 2****,** **Fig. 3****)** appeared crucial to proceed fast and parallelized without being compromised in precision (Wittmann C, Kim HM, Heinzle E: Metabolic network analysis of lysine producing Corynebacterium glutamicum at a miniaturized scale. Biotechnology and bioengineering 2004, 87:1-6. Buchenauer A, Hofmann MC, Funke M, Büchs J, Mokwa W, Schnakenberg U: Micro-bioreactors for fed-batch fermentations with integrated online monitoring and microfluidic devices. Biosensors and Bioelectronics 2009, 24:1411-1416. Kohlstedt M, Starck S, Barton N, Stolzenberger J, Selzer M, Mehlmann K, Schneider R, Pleissner D, Rinkel J, Dickschat JS, et al: From lignin to nylon: Cascaded chemical and biochemical conversion using metabolically engineered Pseudomonas putida. Metabolic Engineering 2018, 47:279-293.) [49-51].

Using a simple batch process, the desired product was finally obtained at 74 g L⁻¹, outcompeting all previous efforts, and exceeding the best titer reported so far, five-fold (**Fig. 7**). For this achievement, the product was transformed from ectoine, additionally adding sugar to support cell growth, a well-accepted process concept in chemical manufacturing (Becker J, Kuhl M, Kohlstedt M, Starck S, Wittmann C: Metabolic engineering of Corynebacterium glutamicum for the production of cis, cis-muconic acid from lignin. Microb Cell Fact 2018, 17:115.) [52], From an industrial perspective, the demonstrated bioconversion strategy appears promising. First, it provides the product of interest at high efficiency. Second, because ectoine is already produced at the multi-ton level, it is available in sufficient amount to be converted into (currently not available) hydroxyectoine at large scale so that the novel product could be explored and commercialized further, including mode-of-action, and safety studies, formulation developments, as well as the preparation and evaluation of product samples (Becker J, Wittmann C: Microbial production of extremolytes - high-value active ingredients for nutrition, health care, and well-being. Current opinion in biotechnology 2020, 65:118-128. Casale M, Moffa A, Carbone S, Fraccaroli F, Costantino A, Sabatino L, Lopez MA, Baptista P, Cassano M, Rinaldi V: Topical Ectoine: A Promising Molecule in the Upper Airways Inflammation-A Systematic Review. BioMed research international 2019, 2019:7150942. Refaat R, Sarhan D, Kotb M, El-Abd E, El-Bassiouni E: Post-irradiation protective effects of ectoine on brain and testicles in male mice. Pharmacological Reports 2018, 70:304-308. Zhang L, Wang Y, Zhang C, Wang Y, Zhu D, Wang C, Nagata S: Supplementation effect of ectoine on thermostability of phytase. Journal of bioscience and bioengineering 2006, 102:560-563.) [1, 53-55]. Production through bioconversion from ectoine, as demonstrated here, seems attractive to open the hydroxyectoine market.

**A two-stage fermentation process with two complementary C. *glutamicum* cell factories appears attractive to produce hydroxyectoine from sugar.** Towards producing hydroxyectoine at even improved economic viability, there is no doubt that a simplified two-stage fermentation process without intermediate purification of ectoine would work as well and might finally display the strategy of choice. To this end, the inventors built on our recently created strain *C*. *glutamicum ectABC^{opt}.* This mutant, also based on *C*. *glutamicum* ATCC 13032, accumulated 65.2 g L⁻¹ of ectoine *de-novo* from sugar (Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.) [15] and thereby favorably approaches the level of 75 g L⁻¹, found optimal for bioconversion in this work (**Fig. 5d****, Table** ). Here, the inventors successfully demonstrated the 2-step bioconversion process is generally doable. With simple cell removal after the ectoine-producing stage, enrichment with fresh sugar, and inoculation with the novel bio-transformation cell factory *C*. *glutamicum P_{tuf}ectD^{MSM},* developed here, hydroxyectoine was produced directly in the broth without need for transient ectoine purification. The inventors show that this two-step concept has huge potential for scale-up and further optimization to industrial scale. Because the two *C*. *glutamicum* strains are identical, except for the heterologous biosynthetic genes, they do not pose any extra demand on the used equipment so that e. g. the same seed line and the same production tank, even the same raw materials, could be used. Compared to direct *de-novo* biosynthesis of hydroxyectoine in one step (Ma Q, Xia L, Wu H, Zhuo M, Yang M, Zhang Y, Tan M, Zhao K, Sun Q, Xu Q, et al: Metabolic engineering of Escherichia coli for efficient osmotic stress-free production of compatible solute hydroxyectoine. Biotechnology and bioengineering 2022, 119:89-101.) [22], such a two-stage strategy would require approximately twice as much sugar. However, given the price of extremolytes of 1,000 USD per kg and more (Liu M, Liu H, Shi M, Jiang M, Li L, Zheng Y: Microbial production of ectoine and hydroxyectoine as high-value chemicals. Microbial Cell Factories 2021, 20:76. Czech L, Hermann L, Stöveken N, Richter AA, Höppner A, Smits SHJ, Heider J, Bremer E: Role of the Extremolytes Ectoine and Hydroxyectoine as Stress Protectants and Nutrients: Genetics, Phylogenomics, Biochemistry, and Structural Analysis. Genes 2018, 9:177.) [5, 19], at least thousand-fold higher than the sugar price (1.1 USD per kg), extra substrate costs appear negligible here (**Global product prices - USA** - **sugar** - **price, March 2022** [https://wvw.globalproductprices.com/USA/sugar_prices/]) [59]. Moreover, at the same time, the double amount of sugar would deliver five-fold more hydroxyectoine (**Fig. 7b**). The metabolic engineering approach that demonstrated *de-novo* biosynthesis of hydroxyectoine in *E. coli* (Ma Q, Xia L, Wu H, Zhuo M, Yang M, Zhang Y, Tan M, Zhao K, Sun Q, Xu Q, et al: Metabolic engineering of Escherichia coli for efficient osmotic stress-free production of compatible solute hydroxyectoine. Biotechnology and bioengineering 2022, 119:89-101.) [22] is only a nice showcase to selectively produce in a difficult pathway constellation. This strategy is less efficient at this stage, and further strain engineering seems problematic as most things are already optimized out.

As shown, metabolically engineered *C. glutamicum* exhibited highly selective production. At small scale, hydroxyectoine was produced at 97% selectivity, while the best-titer process still achieved 78% selectivity. This opens several opportunities. Notably, mixtures of hydroxyectoine and ectoine act as multifunctional anti-pollution agent [60]. Blends of the two molecules have been commercialized by the company bitop under the protected trade name 28Extremoin^{®}. It appears straightforward to directly work up the obtained (roughly) 20:80 mixture of ectoine and hydroxyectoine, obtained here, towards such or similar blends. This would provide a high-value active ingredient at (i) high level, (ii) from a simple defined medium formulation, and (iii) without the need for complex and expensive separation of the two ectoines. The addition of (commercially available) ectoine would allow to adjust other extremolyte mixtures with likely different functional properties. Further optimization of strain and process might help to reach even higher hydroxyectoine levels towards a pure hydroxyectoine product.

### REFERENCES

1. Becker J, Wittmann C: Microbial production of extremolytes - high-value active ingredients for nutrition, health care, and well-being. Current opinion in biotechnology 2020, 65:118-128.
2. Sahle CJ, Schroer MA, Jeffries CM, Niskanen J: Hydration in aqueous solutions of ectoine and hydroxyectoine. Physical Chemistry Chemical Physics 2018, 20:27917-27923.
3. Schwibbert K, Marin-Sanguino A, Bagyan I, Heidrich G, Lentzen G, Seitz H, Rampp M, Schuster SC, Klenk H-P, Pfeiffer F, et al: A blueprint of ectoine metabolism from the genome of the industrial producer Halomonas elongata DSM 2581 T. Environmental microbiology 2011, 13:1973-1994.
4. Pastor JM, Salvador M, Argandoña M, Bernal V, Reina-Bueno M, Csonka LN, Iborra JL, Vargas C, Nieto JJ, Cánovas M: Ectoines in cell stress protection: Uses and biotechnological production. Biotechnology advances 2010, 28:782-801.
5. Liu M, Liu H, Shi M, Jiang M, Li L, Zheng Y: Microbial production of ectoine and hydroxyectoine as high-value chemicals. Microbial Cell Factories 2021, 20:76.
6. Kuhlmann AU, Hoffmann T, Bursy J, Jebbar M, Bremer E: Ectoine and hydroxyectoine as protectants against osmotic and cold stress: uptake through the SigB-controlled betaine-choline- carnitine transporter-type carrier EctT from Virgibacillus pantothenticus. Journal of bacteriology 2011, 193:4699-4708.
7. Smiatek J, Harishchandra RK, Rubner O, Galla H-J, Heuer A: Properties of compatible solutes in aqueous solution. Biophysical chemistry 2012, 160:62-68.
8. Argandoña M, Nieto JJ, Iglesias-Guerra F, Calderón Ml, Garcia-Estepa R, Vargas C: Interplay between iron homeostasis and the osmotic stress response in the halophilic bacterium Chromohalobacter salexigens. Applied and Environmental Microbiology 2010, 76:3575-3589.
9. Graf R, Anzali S, Buenger J, Pfluecker F, Driller H: The multifunctional role of ectoine as a natural cell protectant. Clinics in dermatology 2008, 26:326-333.
10. Kunte HJ, Lentzen G, Galinski E: Industrial Production of the Cell Protectant Ectoine: Protection Mechanisms, Processes, and Products. Current Biotechnology 2014, 3:10-25.
11. Jorge CD, Borges N, Bagyan I, Bilstein A, Santos H: Potential applications of stress solutes from extremophiles in protein folding diseases and healthcare. Extremophiles 2016, 20:251-259.
12. Bownik A, Stepniewska Z: Ectoine as a promising protective agent in humans and animals. Archives of Industrial Hygiene and Toxicology 2016, 67:260-265.
13. Eichel A, Wittig J, Shah-Hosseini K, Mösges R: A prospective, controlled study of SNS01 (ectoine nasal spray) compared to BNO-101 (phytotherapeutic dragées) in patients with acute rhinosinusitis. Current medical research and opinion 2013, 29:739-746.
14. Becker J, Schäfer R, Kohlstedt M, Harder BJ, Borchert NS, Stöveken N, Bremer E, Wittmann C: Systems metabolic engineering of Corynebacterium glutamicum for production of the chemical chaperone ectoine. Microbial Cell Factories 2013, 12:110.
15. Gießelmann G, Dietrich D, Jungmann L, Kohlstedt M, Jeon EJ, Yim SS, Sommer F, Zimmer D, Mühlhaus T, Schroda M, et al: Metabolic Engineering of Corynebacterium glutamicum for High-Level Ectoine Production: Design, Combinatorial Assembly, and Implementation of a Transcriptionally Balanced Heterologous Ectoine Pathway. Biotechnology journal 2019, 14:1800417.
16. Pérez-García F, Ziert C, Risse JM, Wendisch VF: Improved fermentative production of the compatible solute ectoine by Corynebacterium glutamicum from glucose and alternative carbon sources. Journal of Biotechnology 2017, 258:59-68.
17. Ning Y, Wu X, Zhang C, Xu Q, Chen N, Xie X: Pathway construction and metabolic engineering for fermentative production of ectoine in Escherichia coli. Metabolic Engineering 2016, 36:10-18.
18. Lentzen G, Schwarz T: Extremolytes: natural compounds from extremophiles for versatile applications. Applied microbiology and biotechnology 2006, 72:623-634.
19. Czech L, Hermann L, Stöveken N, Richter AA, Höppner A, Smits SHJ, Heider J, Bremer E: Role of the Extremolytes Ectoine and Hydroxyectoine as Stress Protectants and Nutrients: Genetics, Phylogenomics, Biochemistry, and Structural Analysis. Genes 2018, 9:177.
20. Sauer T, Galinski EA: Bacterial milking: A novel bioprocess for production of compatible solutes. Biotechnol Bioeng 1998, 57:306-313.
21. Chen W-C, Hsu C-C, Wang L-F, Lan JC-W, Chang Y-K, Wei Y-H: Exploring useful fermentation strategies for the production of hydroxyectoine with a halophilic strain, Halomonas salina BCRC 17875. Journal of bioscience and bioengineering 2019, 128:332-336.
22. Ma Q, Xia L, Wu H, Zhuo M, Yang M, Zhang Y, Tan M, Zhao K, Sun Q, Xu Q, et al: Metabolic engineering of Escherichia coli for efficient osmotic stress-free production of compatible solute hydroxyectoine. Biotechnology and bioengineering 2022, 119:89-101.
23. Czech L, Stöveken N, Bremer E: EctD-mediated biotransformation of the chemical chaperone ectoine into hydroxyectoine and its mechanosensitive channel-independent excretion. Microbial Cell Factories 2016, 15:126.
24. Hoffmann SL, Kohlstedt M, Jungmann L, Hutter M, Poblete-Castro I, Becker J, Wittmann C: Cascaded valorization of brown seaweed to produce I-lysine and value-added products using Corynebacterium glutamicum streamlined by systems metabolic engineering. Metabolic Engineering 2021, 67:293-307.
25. Kind S, Neubauer S, Becker J, Yamamoto M, Völkert M, Abendroth Gv, Zelder O, Wittmann C: From zero to hero - Production of bio-based nylon from renewable resources using engineered Corynebacterium glutamicum. Metabolic Engineering 2014, 25:113-123.
26. Rohles CM, Gläser L, Kohlstedt M, Gießelmann G, Pearson S, del Campo A, Becker J, Wittmann C: A bio-based route to the carbon-5 chemical glutaric acid and to bionylon-6,5 using metabolically engineered Corynebacterium glutamicum. Green Chemistry 2018, 20:4662-4674.
27. Rohles CM, Pauli S, Giesselmann G, Kohlstedt M, Becker J, Wittmann C: Systems metabolic engineering of Corynebacterium glutamicum eliminates all by-products for selective and high-yield production of the platform chemical 5-aminovalerate. Metabolic Engineering 2022, 73:168-181.
28. Wittmann C, Heinzle E: Modeling and experimental design for metabolic flux analysis of lysine-producing corynebacteria by mass spectrometry. Metab Eng 2001, 3:173-191.
29. Becker J, Klopprogge C, Zelder O, Heinzle E, Wittmann C: Amplified Expression of Fructose 1,6-Bisphosphatase in Corynebacterium glutamicum Increases In Vivo Flux through the Pentose Phosphate Pathway and Lysine Production on Different Carbon Sources. Applied and Environmental Microbiology 2005, 71:8587-8596.
30. Becker J, Zelder O, Häfner S, Schröder H, Wittmann C: From zero to hero-Design-based systems metabolic engineering of Corynebacterium glutamicum for I-lysine production. Metabolic Engineering 2011, 13:159-168.
31. Bursy J, Pierik AJ, Pica N, Bremer E: Osmotically Induced Synthesis of the Compatible Solute Hydroxyectoine Is Mediated by an Evolutionarily Conserved Ectoine Hydroxylase. Journal of Biological Chemistry 2007, 282:31147-31155.
32. Widderich N, Pittelkow M, Höppner A, Mulnaes D, Buckel W, Gohlke H, Smits SHJ, Bremer E: Molecular Dynamics Simulations and Structure-Guided Mutagenesis Provide Insight into the Architecture of the Catalytic Core of the Ectoine Hydroxylase. Journal of molecular biology 2014, 426:586-600.
33. Ofer N, Wishkautzan M, Meijler M, Wang Y, Speer A, Niederweis M, Gur E: Ectoine biosynthesis in Mycobacterium smegmatis. Applied and Environmental Microbiology 2012, 78:7483-7486.
34. Bursy J, Kuhlmann AU, Pittelkow M, Hartmann H, Jebbar M, Pierik AJ, Bremer E: Synthesis and uptake of the compatible solutes ectoine and 5-hydroxyectoine by Streptomyces coelicolor A3(2) in response to salt and heat stresses. Applied and Environmental Microbiology 2008, 74:7286-7296.
35. Leszczewicz M, Walczak P: Selection of Thermotolerant Corynebacterium glutamicum Strains for Organic Acid Biosynthesis. Food Technology and Biotechnology 2019, 57:249-259.
36. Widderich N, Höppner A, Pittelkow M, Heider J, Smits SHJ, Bremer E: Biochemical properties of ectoine hydroxylases from extremophiles and their wider taxonomic distribution among microorganisms. PloS one 2014, 9:e93809.
37. Kushwaha B, Jadhav I, Verma HN, Geethadevi A, Parashar D, Jadhav K: Betaine accumulation suppresses the de-novo synthesis of ectoine at a low osmotic concentration in Halomonas sp SBS 10, a bacterium with broad salinity tolerance. Molecular biology reports 2019, 46:4779-4786.
38. Wittmann C, Weber J, Betiku E, Krömer J, Bohm D, Rinas U: Response of fluxome and metabolome to temperature-induced recombinant protein synthesis in Escherichia coli. J Biotechnol 2007, 132:375-384.
39. Kohlstedt M, Sappa PK, Meyer H, Maaß S, Zaprasis A, Hoffmann T, Becker J, Steil L, Hecker M, van Dijl JM, et al: Adaptation of Bacillus subtilis carbon core metabolism to simultaneous nutrient limitation and osmotic challenge: a multi-omics perspective. Environmental microbiology 2014, 16:1898-1917.
40. Smiatek J, Harishchandra RK, Galla H-J, Heuer A: Low concentrated hydroxyectoine solutions in presence of DPPC lipid bilayers: A computer simulation study. Biophysical chemistry 2013, 180-181:102-109**.**
41. Smiatek J: Osmolyte Effects: Impact on the Aqueous Solution around Charged and Neutral Spheres. The Journal of Physical Chemistry B 2014, 118:771-782.
42. Herzog M, Dwivedi M, Kumar Harishchandra R, Bilstein A, Galla H-J, Winter R: Effect of ectoine, hydroxyectoine and β-hydroxybutyrate on the temperature and pressure stability of phospholipid bilayer membranes of different complexity. Colloids and Surfaces B: Biointerfaces 2019, 178:404-411.
43. Becker J, Rohles CM, Wittmann C: Metabolically engineered Corynebacterium glutamicum for bio-based production of chemicals, fuels, materials, and healthcare products. Metabolic Engineering 2018, 50:122-141.
44. Wolf S, Becker J, Tsuge Y, Kawaguchi H, Kondo A, Marienhagen J, Bott M, Wendisch Volker F, Wittmann C: Advances in metabolic engineering of Corynebacterium glutamicum to produce high-value active ingredients for food, feed, human health, and well-being. Essays in Biochemistry 2021, 65:197-212.
45. Weiland F, Kohlstedt M, Wittmann C: Guiding stars to the field of dreams: Metabolically engineered pathways and microbial platforms for a sustainable lignin-based industry. Metabolic Engineering 2022, 71:13-41.
46. Kind S, Jeong WK, Schröder H, Wittmann C: Systems-wide metabolic pathway engineering in Corynebacterium glutamicum for bio-based production of diaminopentane. Metabolic Engineering 2010, 12:341-351.
47. Kind S, Kreye S, Wittmann C: Metabolic engineering of cellular transport for overproduction of the platform chemical 1,5-diaminopentane in Corynebacterium glutamicum. Metabolic Engineering 2011, 13:617-627.
48. Rohles CM, Gießelmann G, Kohlstedt M, Wittmann C, Becker J: Systems metabolic engineering of Corynebacterium glutamicum for the production of the carbon-5 platform chemicals 5-aminovalerate and glutarate. Microbial Cell Factories 2016, 15:154.
49. Wittmann C, Kim HM, Heinzle E: Metabolic network analysis of lysine producing Corynebacterium glutamicum at a miniaturized scale. Biotechnology and bioengineering 2004, 87:1-6.
50. Buchenauer A, Hofmann MC, Funke M, Büchs J, Mokwa W, Schnakenberg U: Micro-bioreactors for fed-batch fermentations with integrated online monitoring and microfluidic devices. Biosensors and Bioelectronics 2009, 24:1411-1416.
51. Kohlstedt M, Starck S, Barton N, Stolzenberger J, Selzer M, Mehlmann K, Schneider R, Pleissner D, Rinkel J, Dickschat JS, et al: From lignin to nylon: Cascaded chemical and biochemical conversion using metabolically engineered Pseudomonas putida. Metabolic Engineering 2018, 47:279-293.
52. Becker J, Kuhl M, Kohlstedt M, Starck S, Wittmann C: Metabolic engineering of Corynebacterium glutamicum for the production of cis, cis-muconic acid from lignin. Microb Cell Fact 2018, 17:115.
53. Casale M, Moffa A, Carbone S, Fraccaroli F, Costantino A, Sabatino L, Lopez MA, Baptista P, Cassano M, Rinaldi V: Topical Ectoine: A Promising Molecule in the Upper Airways Inflammation-A Systematic Review. BioMed research international 2019, 2019:7150942.
54. Refaat R, Sarhan D, Kotb M, El-Abd E, El-Bassiouni E: Post-irradiation protective effects of ectoine on brain and testicles in male mice. Pharmacological Reports 2018, 70:304-308.
55. Zhang L, Wang Y, Zhang C, Wang Y, Zhu D, Wang C, Nagata S: Supplementation effect of ectoine on thermostability of phytase. Journal of bioscience and bioengineering 2006, 102:560-563.
56. Xue C, Hsu K-M, Ting W-W, Huang S-F, Lin H-Y, Li S-F, Chang J-S, Ng IS: Efficient biotransformation of I-lysine into cadaverine by strengthening pyridoxal 5'-phosphate-dependent proteins in Escherichia coli with cold shock treatment. Biochemical Engineering Journal 2020, 161:107659.
57. Cheng J, Tu W, Luo Z, Gou X, Li Q, Wang D, Zhou J: A High-Efficiency Artificial Synthetic Pathway for 5-Aminovalerate Production From Biobased L-Lysine in Escherichia coli. Frontiers in Bioengineering and Biotechnology 2021, 9:633028.
58. Shi X, Chang C, Ma S, Cheng Y, Zhang J, Gao Q: Efficient bioconversion of I-glutamate to γ-aminobutyric acid by Lactobacillus brevis resting cells. Journal of Industrial Microbiology and Biotechnology 2017, 44:697-704.
59. Global product prices - USA - sugar - price, March 2022 [https://www.globalproductprices.com/USA/sugar prices/]
60. Bilstein A, Krutmann J: Solute and solute mixture, and a composition containing at least one solute, for use in the prevention or treatment of cosmetic or pathological efflorescences caused by particulate matter vol. WO2016045714A1: bitop AG; 2016.
61. Gómez-Álvarez H, Iturbe P, Rivero-Buceta V, Mines P, Bugg TDH, Nogales J, Diaz E: Bioconversion of lignin-derived aromatics into the building block pyridine 2,4-dicarboxylic acid by engineering recombinant Pseudomonas putida strains. Bioresource technology 2022, 346:126638.
62. Eikmanns BJ: Central metabolism: tricarboxylic acid cycle and anapierotic reactions. In Handbook of Corynebacterium glutamicum. Edited by Eggeling L, Bott M. Boca Raton: CRC Press; 2005: 241-276.
63. Wen J, Bao J: Engineering Corynebacterium glutamicum triggers glutamic acid accumulation in biotin-rich corn stover hydrolysate. Biotechnology for Biofuels 2019, 12:86.
64. Nguyen AQD, Schneider J, Reddy GK, Wendisch VF: Fermentative production of the diamine putrescine: system metabolic engineering of Corynebacterium glutamicum. Metabolites 2015, 5:211-231.
65. Kempf B, Bremer E: Uptake and synthesis of compatible solutes as microbial stress responses to high-osmolality environments. Archives of Microbiology 1998, 170:319-330.
66. Oren A: Bioenergetic Aspects of Halophilism. Microbiology and Molecular Biology Reviews 1999, 63:334-348.
67. Bestvater T, Louis P, Galinski EA: Heterologous ectoine production in Escherichia coli: by-passing the metabolic bottle-neck. Saline systems 2008, 4:12.
68. Tanne C, Golovina EA, Hoekstra FA, Meffert A, Galinski EA: Glass-forming property of hydroxyectoine is the cause of its superior function as a desiccation protectant. Frontiers in Microbiology 2014, 5:150.
69. Empadinhas N, da Costa MS: Osmoadaptation mechanisms in prokaryotes: distribution of compatible solutes. International microbiology: the official journal of the Spanish Society for Microbiology 2008, 11:151-161.
70. Wood JM, Bremer E, Csonka LN, Kraemer R, Poolman B, van der Heide T, Smith LT: Osmosensing and osmoregulatory compatible solute accumulation by bacteria. Comparative Biochemistry and Physiology Part A: Molecular & Integrative Physiology 2001, 130:437-460.
71. Frings E, Kunte HJ, Galinski EA: Compatible solutes in representatives of the genera Brevibacterium and Corynebacterium: Occurrence of tetrahydropyrimidines and glutamine. FEMS Microbiology Letters 1993, 109:25-32.
72. Salvador M, Argandoña M, Naranjo E, Piubeli F, Nieto JJ, Csonka LN, Vargas C: Quantitative RNA-seq Analysis Unveils Osmotic and Thermal Adaptation Mechanisms Relevant for Ectoine Production in Chromohalobacter salexigens. Frontiers in microbiology 2018, 9:1845**.**
73. Ferreira C, Soares AR, Lamosa P, Santos MA, da Costa MS: Comparison of the compatible solute pool of two slightly halophilic planctomycetes species, Gimesia marts and Rubinisphaera brasiliensis. Extremophiles 2016, 20:811-820.
74. Garcia-Estepa R, Cánovas D, Iglesias-Guerra F, Ventosa A, Csonka LN, Nieto JJ, Vargas C: Osmoprotection of Salmonella enterica serovar Typhimurium by Nγ-acetyldiaminobutyrate, the precursor of the compatible solute ectoine. Systematic and applied microbiology 2006, 29:626-633.
75. Steger R, Weinand M, Krämer R, Morbach S: LcoP, an osmoregulated betaine/ectoine uptake system from Corynebacterium glutamicum. FEBS letters 2004, 573:155-160.
76. Buschke N, Schröder H, Wittmann C: Metabolic engineering of Corynebacterium glutamicum for production of 1,5-diaminopentane from hemicellulose. Biotechnology journal 2011, 6:306-317.
77. Yim SS, Choi JW, Lee RJ, Lee YJ, Lee SH, Kim SY, Jeong KJ: Development of a new platform for secretory production of recombinant proteins in Corynebacterium glutamicum. Biotechnology and Bioengineering 2016, 113:163-172.
78. Gibson DG, Young L, Chuang R-Y, Venter JC, Hutchison Iii CA, Smith HO: Enzymatic assembly of DNA molecules up to several hundred kilobases. Nature Methods 2009, 6:343-345.
79. Becker J, Buschke N, Bücker R, Wittmann C: Systems level engineering of Corynebacterium glutamicum - Reprogramming translational efficiency for superior production. Engineering in Life Sciences 2010, 10:430-438.
80. Becker J, Klopprogge C, Wittmann C: Metabolic responses to pyruvate kinase deletion in lysine producing Corynebacterium glutamicum. Microbial Cell Factories 2008, 7:8.
81. Bolten CJ, Kiefer P, Letisse F, Portais J-C, Wittmann C: Sampling for Metabolome Analysis of Microorganisms. Analytical chemistry 2007, 79:3843-3849.
82. Hans MA, Heinzle E, Wittmann C: Quantification of intracellular amino acids in batch cultures of Saccharomyces cerevisiae. Appl Microbiol Biotechnol 2001, 56:776-779.
83. Hara R, Nishikawa T, Okuhara T, Koketsu K, Kino K: Ectoine hydroxylase displays selective trans-3-hydroxylation activity towards I-proline. Applied microbiology and biotechnology 2019, 103:5689-5698**.**
84. Krömer J, Fritz M, Heinzle E, Wittmann C: In vivo quantification of intracellular amino acids and intermediates of the methionine pathway in Corynebacterium glutamicum. Analytical biochemistry 2005, 340:171-173.
85. Buschke N, Becker J, Schäfer R, Kiefer P, Biedendieck R, Wittmann C: Systems metabolic engineering of xylose-utilizing Corynebacterium glutamicum for production of 1,5-diaminopentane. Biotechnology journal 2013, 8:557-570.
86. Bethlehem L, Moritz KD: Boosting Escherichia coli's heterologous production rate of ectoines by exploiting the non-halophilic gene cluster from Acidiphilium cryptum. Extremophiles 2020, 24:733-747.
87. Eilert E, Kranz A, Hollenberg CP, Piontek M, Suckow M: Synthesis and release of the bacterial compatible solute 5-hydroxyectoine in Hansenula polymorpha. Journal of Biotechnology 2013, 167:85-93.

## Claims

1. A method for producing hydroxyectoine, comprising
(a) providing a biocatalyst, wherein said biocatalyst is a recombinant bacterial host cell transformed with a promoter operably linked to a nucleotide sequence encoding an ectoine hydroxylase - ectD - (EC 1.14.11) followed by a transcriptional terminator,
(b) providing a carbon source and ectoine, and
(c) cultivating the bacterial host cell under suitable conditions to allow biocatalytic conversion of ectoine into hydroxyectoine, and thereby secreting the produced hydroxyectoine.

2. The method of claim 1, wherein the nucleotide sequence encoding the ectoine hydroxylase is selected from the group consisting of *Pseudomonas stutzeri, Mycobacterium smegmatis, Halomonas elongata, Virgibacillus salexigens, Streptomyces coelicolor, Gracilibacillus sp., Acidiphilium cryptum, Alkalihalobacillus clausii, Hydrocarboniclastica marina, Methylomicrobium alcaliphilum, Neptunomonas concharum, Leptospirillum ferriphilum, Candidatus Nitrosopumilus sp., Sphyngopyxis alaskensis, Paenibacillus lautus,* and *Chromohalobacter salexigens.*

3. The method of claim 1 or 2, wherein said bacterial host cell is generally recognized as safe (GRAS) bacterial host cell, preferably wherein said bacterial host cell is selected from the group consisting of *Actinobacteria,* preferably *Actinobacteridae,* more preferably *Actinomycetales,* even more preferred *Corynebacterineae,* in particular preferred *Corynebacterium* ssp., most preferred *Corynebacterium glutamicum.*

4. The method of any one of the preceding claims, wherein the method is suitable to be conducted in batch mode or fed batch mode; preferably wherein said bacterial host cell is capable of producing hydroxyectoine at a concentration in the range of about 20 g L⁻¹ to about 100 g L⁻¹, preferably of about 30 g L⁻¹ to 90 g L⁻¹, more preferably of about 40 g L⁻¹ to about 80 g L⁻¹, most preferably of about 75 g L⁻¹; wherein said bacterial host cell is capable of producing hydroxyectoine within a period of time selected from about 24 h, about 20 h, about 18 h, about 15 h, about 10 h, about 12 h, and about 8 h; preferably wherein said bacterial host cell is capable of producing hydroxyectoine with a productivity of about 1.5 g/L/h to about 8 g/L/h, preferably of about 3 g/L/h to about 7.5 g/L/h, more preferably of about 4 g/L/h to about 7 g/L/h, most preferably of about 6.25 g/L/h.

5. The method of any one of the preceding claims, wherein the promoter is a constitutive promoter, and wherein the promoter is a homologous or heterologous promoter, and preferably selected from the group consisting of P_{tuf}, P_{sod} (Superoxiddismutase), P_{cspB} (surface-layer protein PS2), and P_{gapA} (glyceraldehyde 3-phosphate dehydrogenase; preferably wherein the promoter operably linked to a nucleotide sequence encoding an ectoine hydroxylase - ectD - (EC 1.14.11) is comprised in a plasmid, preferably an episomal plasmid, preferably a plasmid that can replicate in *C*. *glutamicum,* wherein the plasmid is preferably selected from pClik and pCES.

6. The method of any one of the preceding claims, wherein the cultivation of the bacterial host cell in step (c) is conducted at temperatures of about 25°C to about 45°C, preferably between about 30°C to about 37°C.

7. The method of any one of the preceding claims, wherein the cultivation of the bacterial host cell in step (c) is conducted comprising the provision of the ectoine at a concentration of about 40 g L⁻¹ to about 120 g L⁻¹ and providing the carbon source at concentration of about 90 g L⁻¹ to about 220 g L⁻¹.

8. The method of any one of the preceding claims, wherein instead of or additionally to step (b) the method comprises
(b1) providing a further biocatalyst, wherein said further biocatalyst is a further bacterial host cell transformed with a heterologous promoter operably linked to a nucleotide sequence encoding
(i) a heterologous promoter operably linked to a nucleotide sequence encoding 2,4-diaminobutyrate acetyltransferase (EC 2.3.1.178) followed by a transcriptional terminator,
(ii) a heterologous promoter operably linked to a nucleotide sequence encoding L-2,4-diaminobutyrate transaminase (EC 2.6.1.76) followed by a transcriptional terminator, and
(iii) a heterologous promoter operably linked to a nucleotide sequence encoding ectoine synthase (EC 4.2.1.108) followed by a transcriptional terminator;
(b2) providing carbon source,
(b3) cultivating said further bacterial host cell under suitable conditions to allow biocatalytic conversion of glucose into ectoine, and preferably,
(b4) separating of the further bacterial host cell.

9. The method of any one of the preceding claims, wherein said further bacterial host cell is generally recognized as safe (GRAS) bacterial host cell; preferably wherein said further bacterial host cell is selected from the group consisting of *Corynebacterium* ssp., preferably *Corynebacterium glutamicum, Escherichia coli, Halomonas elongata, Chromohalobacter salexigens,* and *Bacillus subtilis.*

10. The method of any one of the preceding claims, wherein said nucleotide sequence encoding an ectoine hydroxylase - ectD - (EC 1.14.11) is selected from the group consisting of SEQ ID NO:7 to 38; preferably wherein said nucleotide sequence encoding 2,4-diaminobutyrate acetyltransferase (EC 2.3.1.178) is selected from SEQ ID NO:4; preferably wherein said nucleotide sequence encoding L-2,4-diaminobutyrate transaminase (EC 2.6.1.76) is selected from SEQ ID NO:5; preferably wherein said nucleotide sequence encoding ectoine synthase (EC 4.2.1.108) is selected from SEQ ID NO:6.

11. The method of any one of the preceding claims, wherein the method further comprises after step (c)
(d) breaking up of said bacterial host cell to recover also intracellularly produced hydroxyectoine.

12. A recombinant bacterial host cell comprising a promoter operably linked to a nucleotide sequence encoding an ectoine hydroxylase - ectD - (EC 1.14.11) followed by a transcriptional terminator; preferably wherein said host cell comprises a promoter as shown in SEQ ID NO:3 operably linked to a nucleotide sequence encoding an ectoine hydroxylase - ectD - (EC 1.14.11) selected from the group consisting of SEQ ID NO:7 to 38 followed by a transcriptional terminator.

13. The recombinant bacterial host cell of claim 12, wherein said bacterial host cell is capable of producing hydroxyectoine at a concentration in the range of about 20 g L⁻¹ to about 100 g L⁻¹, preferably in the range of about 30 g L⁻¹ to 90 g L⁻¹, more preferably of about 40 g L⁻¹ to about 80 g L⁻¹, most preferably of about 75 g L⁻¹; preferably wherein said bacterial host cell is capable of producing hydroxyectoine within a period of time selected from about 24 h, about 20 h, about 18 h, about 15 h, about 10 h, about 12 h, and about 8 h; preferably wherein said bacterial host cell is capable of producing hydroxyectoine with a productivity of about 1.5 g/L/h to about 8 g/L/h, preferably of about 3 g/L/h to about 7.5 g/L/h, more preferably of about 4 g/L/h to about 7 g/L/h, most preferably of about 6.25 g/L/h.

14. Use of the recombinant bacterial host cell of claim 12 or 13 for the production of hydroxyectoine.

15. Kit comprising a bacterial host cell of claim 12 or 13 and a further bacterial host cell as defined in any one of claims 8 to 10 for use in a method of any one of the claims 1 to 11.
